# EUROPEAN PATENT SPECIFICATION

(11) **EP 3 317 404 B1**
(45) Date of publication and mention of the grant of the patent: **28.04.2021**
(21) Application number: 16733583.5
(22) Date of filing: 30.06.2016
(51) Int. Cl.: C12N 5/079, C12N 5/00

(54) **CULTURE OF RPE CELLS**
KULTIVIERUNG VON RPE-ZELLEN
CULTURE DE CELLULES RPE

(30) Priority: 30.06.2015 GB 201511482
(43) Date of publication of application: 09.05.2018
(73) Proprietor: Oslo Universitetssykehus HF, 0424 Oslo (NO)
(72) Inventor: EIDET, Jon Roger, 0484 Oslo (NO); UTHEIM, Tor, 0286 Oslo (NO); REPPE, Sjur, 2004 Lillestrøm (NO); LYBERG, Torstein, 0787 Oslo (NO)
(74) Representative: Dehns
(86) International application number: PCT/EP2016/065357
(87) International publication number: WO 2017/001589

(56) References cited:
- JP-A- 2011 152 111
- US-A1- 2005 143 296
- US-A1- 2013 149 284
- TERADA S ET AL: "Preparation of silk protein sericin as mitogenic factor for better mammalian cell culture", JOURNAL OF BIOSCIENCE AND BIOENGINEERING, ELSEVIER, AMSTERDAM, NL, vol. 100, no. 6, 1 December 2005 (2005-12-01), pages 667-671, XP027707273, ISSN: 1389-1723 [retrieved on 2005-12-01] cited in the application
- SATOSHI TERADA ET AL: "Sericin, a protein derived from silkworms, accelerates the proliferation of several mammalian cell lines including a hybridoma", CYTOTECHNOLOGY, KLUWER ACADEMIC PUBLISHERS, DO, vol. 40, no. 1-3, 1 November 2002 (2002-11-01), pages 3-12, XP019236747, ISSN: 1573-0778, DOI: 10.1023/A:1023993400608 cited in the application
- J. R. EIDET ET AL: "The Silk-protein Sericin Induces Rapid Melanization of Cultured Primary Human Retinal Pigment Epithelial Cells by Activating the NF-[kappa]B Pathway", SCIENTIFIC REPORTS, vol. 6, 4 March 2016 (2016-03-04), page 22671, XP055291068, DOI: 10.1038/srep22671

## Description

The present invention lies generally in the field of culture of mammalian cells and tissue, notably cells or tissue intended for transplantation. In particular the invention relates to the culture of retinal pigment epithelium (RPE) cells or RPE precursor cells, and more particularly to the use of sericin in the culture medium for RPE cells or RPE precursor cells in order to promote pigmentation, and desirably also differentiation, of the cultured RPE or RPE precursor cells.

Age-related macular degeneration (AMD) is one of the world's leading causes of blindness and impairment or loss of the retinal pigment epithelium (RPE) is a central pathogenic factor. Age-related macular degeneration is subdivided into a dry and a wet type, and there are no established curative treatment options for the dry type of AMD, which constitutes more than 85% of cases. Replacement of the RPE in patients, involving the culture and implantation of RPE transplants, is a promising avenue for treatment of AMD, and several studies have shown the capability of this approach to improve visual function. However, although improvements have been made in technologies for the preparation and culture of cells for cell replacement, the preparation of cells or tissue that fulfill the requirements for transplantation is complex, and refined and improved ways of producing cells such as RPE cells for cell replacement therapies are needed. Indeed, with recent advances of RPE tissue engineering approaches, the need for improved cell culture methods for producing RPE is anticipated to increase in coming years.

For transplantation it is desirable that the transplanted cells retain the characteristics and functional properties of the native cells or tissue they are intended to replace or supplement, i.e. that the transplanted cells are as close as possible to how they appear in nature, or *in situ* in the body (*in vivo*)*.* However, maintaining a native RPE phenotype *in vitro* has proved challenging and it has been observed that RPE cells in culture may de-differentiate or lose the characteristics of native RPE cells. This is particularly true after repeated passages. In particular, the cells of the native RPE are pigmented - melanin is visible in melanin granules in the cells. However, in culture pigmentation may be lost. This is seen particularly in RPE cell lines such as ARPE-19 (see further below), most notably in the available higher passages of this cell line which typically are amelanotic, unless grown in specialised growth media and conditions. However, depigmentation and other aspects of de-differentiation (e.g. loss of RPE cell markers and/or polarity markers, or reduced transepithelial resistance (TER)) may also be observed in primary cultures.

Several culture protocols for producing functional RPE cells have been described from both human embryonic stem cells (hESC) and human induced pluripotent stem cells (iPSC). Such methods are disclosed, for example, in US 2013/0149284. This document teaches methods for producing RPE cells from several different types of human stem cells, including hESCs and iPSCs. Various methods of culturing the obtained RPE cells are also disclosed, including using a specialised RPE Maintenance Medium (RPE-MM) and RPE Growth Medium (RPE-GM).

However, although protocols using hESC or iPSC may successfully produce differentiated and pigmented RPE cells, they are usually time-consuming, sometimes necessitating a total culture period of up to 90 days. Cutting down the time necessary for producing differentiated RPE reduces the costs of production. It would also lessen the risk of infection that may occur during prolonged cell culturing. Thus, for further advancement of RPE replacement therapies, the establishment of alternative and improved culture techniques for producing RPE cells, and in particular RPE cells which better retain RPE characteristics or in which RPE characteristics may be restored, would be advantageous.

The present invention is directed to meeting this need, and in particular to the goal of providing a standardized, ideally xenobiotic-free, culture protocol for RPE culture in regenerative medicine. In work leading up to the present invention the inventors have found that, unexpectedly, cell culture media for RPE cells may be improved by adding the silk protein sericin to the culture media, and in particular that the inclusion of sericin in the media may promote pigmentation of cultured RPE cells. This was very surprising, as it has previously been reported that sericin inhibits the enzyme tyrosinase, which is an important enzyme required in the process of pigmentation of RPE cells (see e.g. Kato et al. (1998 Biosci. Biotechnol. Biochem. 62:145-147). Indeed, tyrosinase is the only rate-limiting pigmentation enzyme, and thus the inclusion of a tyrosinase inhibitor such as sericin in a medium for culture of RPE cells would have been expected to inhibit or reduce pigmentation. The study in Kato *et al.* demonstrated that sericin inhibits the activity of tyrosinase, which is responsible for melanin production (melanogenesis) in RPE cells. Tyrosinase catalyses the formation of dihydroxyphenylalanine (L-DOPA) from L-tyrosine, and L-DOPA is subsequently converted to melanin, aided by tyrosinase-related proteins 1 and 2 (TRP-1 and TRP-2). Thus, it was unexpected that a medium containing sericin could be used successfully to culture pigmented RPE cells. However, as demonstrated in the Examples below, the present inventors found that the addition of sericin to culture media appeared to promote melanogenesis and induced pigmentation of the cultured cells; pigmentation was not seen in cells cultured in the same media in the absence of sericin. Further, the results show improved differentiation of RPE cells in the presence of sericin in the culture media, as evidenced by expression of RPE cell and polarity markers. This then has led to the proposal, according to the present invention, that sericin may be used to promote pigmentation of RPE cells in culture, and in particular to promote pigmentation and differentiation of RPE cells in culture.

Accordingly, in one aspect the present invention provides use of sericin in the culture of RPE cells or RPE precursor cells, wherein sericin is added to the culture medium and promotes pigmentation of cultured RPE cells or RPE precursor cells.

In particular, the sericin may promote differentiation, including pigmentation, of RPE precursor cells into RPE cells.

Alternatively expressed, this aspect of the invention provides use of sericin as a culture medium additive (or supplement) in the culture of RPE cells or RPE precursor cells to promote pigmentation of cultured RPE cells or RPE precursor cells.

In a further aspect, the present invention also provides a method for the culture of RPE cells or RPE precursor cells, said method comprising culturing said cells in a medium comprising sericin, wherein the sericin promotes pigmentation of the cultured RPE cells or RPE precursor cells.

Alternatively expressed, this aspect of the invention provides a method for promoting pigmentation of RPE cells or RPE precursor cells in culture, said method comprising culturing RPE cells or RPE precursor cells in the presence of sericin.

Essentially, therefore, the inventors have developed an improved culture medium for the culture of RPE cells or RPE precursor cells which allows an improved RPE phenotype to be obtained in the cultured cells, in particular a phenotype which is pigmented (or has improved or enhanced pigmentation) and which preferably also exhibits an improvement or enhancement in other aspects of RPE cell differentiation, namely in which RPE cell differentiation is also enhanced or promoted.

In particular, where the cultured cells are RPE precursor cells the differentiation of the precursor cells into RPE cells may be induced, promoted or improved.

In addition, the inclusion of sericin in the culture medium according to the use and method of the invention facilitates an additional advantage, in that a serum-free medium can be employed. Whilst, in view of its cell survival and growth promoting effects, serum is commonly used for the culture of cells, including RPE cells, it is associated with a number of disadvantages, including potential transmission of animal pathogens, batch-to-batch variability and the risk of immune-rejection due to introduction of animal proteins in a transplant. Sericin has previously been proposed as a serum-replacement in cell culture methods (e.g. in the culture of pancreatic islets). However, in view of its inhibitory effects on tyrosinase, it would not have been thought that it was suitable or desirable to use sericin in a medium for culturing RPE cells whilst preserving or obtaining a pigmented phenotype. Advantageously, the present invention allows the use of a medium for the culture of RPE cells which is serum-free. The present Examples demonstrate the successful culture of RPE cells in serum-free medium containing sericin. As such, in one preferred embodiment of the invention the medium is serum-free, i.e. the invention provides use of a medium for culture of RPE cells, wherein said medium comprises sericin and wherein said medium is serum-free.

"Serum-free" is a term known and recognised in the art in the context of media for cells and tissues, and means simply that the medium does not contain any serum additive or component, that is no additive or component comprising or consisting of serum. Particularly in this context is meant serum from blood. In particular the medium does not contain fetal bovine serum (FBS), fetal calf serum (FCS) or any animal serum or serum product used or proposed for use in media for cells or tissues.

However, in an alternative embodiment the medium may comprise serum, for example FBS or FCS, or any suitable serum. Serum may be present for example at 0.01 to 10 % (w/v), or any one of 0.01, 0.05, 0.1. 0.2, or 0.5 to any one of 10, 8, 6, 5, 4, 3 or 2 %, more particularly 0.1 to 5, 0.1 to 4, 0.1 to 3, 0.1 to 2 % (w/v) (i.e. wherein 1 % serum = 10 mg/mL). In a representative embodiment serum is added at 1 % (w/v). Whilst the avoidance of serum is preferred where possible, there may be circumstances where the inclusion of serum may be desired and hence this is included within the scope of the invention. Indeed, in view of its earlier-proposed use as a serum-replacement, it would be counter-intuitive to include both serum and sericin in a cell culture medium. Such a cell culture medium, for culture of RPE cells and comprising serum and sericin, is disclosed herein. In particular, such a medium may comprise a minimal medium, e.g. a minimum essential medium, as discussed further below, to which sericin and serum are added. Serum can be obtained from any suitable source. Suitable sources are known in the art.

Cell "culture" refers generally to the growth of cells under controlled conditions, generally outside of their natural environment. Thus typically cell culture refers to the *in vitro* or *ex vivo* growth of cells or tissue isolated from their/its natural environment, specifically in this case to RPE cells or tissue removed from the body, or more particularly from the eye. Generally, cell culture takes place under growth-promoting conditions, that is conditions which are conducive or permissive to cell growth (i.e. which are designed or selected to promote or stimulate cell growth) and growth of the cells is typically seen.

"Cell growth" refers to cell proliferation or cell division, or an increase in the number of cells. Thus, the present invention can be employed when it is desirable to increase the number or density of RPE cells, for example for transplantation purposes.

Cell growth and cell density can be determined by standard methods known in the art, for example as used in the Examples below. Thus, cell density may be quantified by staining cell nuclei with DAPI stain and counting cells using light microscopy, for example using the ImageJ software as described herein, or any suitable method.

The present invention is based on the surprising effect that sericin may promote pigmentation of RPE cells in culture. "Promotion of pigmentation" refers generally to any effect in which pigmentation of the cultured RPE cells or RPE precursor cells is improved or enhanced, stimulated or induced. Pigmentation refers to the presence of the pigment melanin in the RPE cells, generally in so-called melanin, or pigment, granules. Promotion of pigmentation may be therefore alternatively be referred to as promotion of melanization or as enhancing the growth of melanotic RPE cells. Included therefore is an increase in the amount of pigment (melanin) in the cultured cells (e.g. the amount of pigment which may be seen, visualised or detected), or an increase in the number and/or size of melanin granules. A native pigmented RPE may generally be seen visually (e.g. microscopically) to contain melanin and promotion of pigmentation includes the maintenance or retention of such a pigmented phenotype or to the induction or development of a pigmented phenotype (or of melanotic RPE cells). Indeed the present Example shows that the presence of visualisable or detectable pigment is induced in initially amelanotic cells cultured in the presence of sericin, whereas pigment in absent (or none is seen or detected) in cells cultured in the absence of sericin. Promotion of pigmentation may thus include an effect of inducing melanogenesis, including in cells in which no pigment is present or detectable prior to culture according to the invention. The present invention may thus be used to induce or stimulate, or to augment or enhance a pigmented phenotype in cells which have become depigmented (i.e. which have lost the pigmented phenotype or in which the amount of pigment is reduced as compared to a normal healthy native RPE). Promotion of pigmentation therefore also includes restoration of a pigmented phenotype or restoration of normal or native levels of pigmentation.

Further, the invention may also be used for the culture of RPE precursor cells, including stem cells (see further below), with the aim of inducing RPE cell differentiation, including pigmentation, i.e. inducing, facilitating, promoting or improving the development of differentiated RPE cells, or of an RPE cell phenotype.

The present invention may thus be used in the culture of both primary cultures of RPE cells, or of established RPE cell cultures or RPE cell lines, in order to maintain (or retain) a pigmented RPE phenotype, or to augment or improve (increase) pigmentation in cultures of RPE cells e.g. of later passages of RPE cell cultures, which have lost or reduced pigmentation as a result of cell culture, or which are at risk of depigmentation. It may also be used for the culture of cells which have become amelanotic or less pigmented, with the aim of restoring pigmentation in the cultured cells. Thus, the invention may be of use in the culture of RPE cell lines such as the ARPE-19 cell line, the later passages of which (p20 onwards) are generally regarded as amelanotic and which are the passages which are now generally available, to restore or enhance pigmentation in such cell lines (i.e. to induce repigmentation).

The results reported below also show that in addition to improved pigmentation other aspects of RPE cell differentiation may also be improved by culture in the presence of sericin. The invention may therefore also include promoting the differentiation of RPE cells.

Cell "differentiation" generally refers to a less specialised cell becoming more specialised. In the context of RPE, differentiation implies the development of the features, characteristics and properties which characterise or identify the cell as an RPE cell, and in particular the full repertoire of features and properties which define or characterise a fully differentiated and mature native RPE cell. Accordingly, sericin may be used to increase maturation of RPE cells by promoting melanogenesis and the visual cycle. As noted above culture of RPE cells, including of primary cultures, may result in de-differentiation and loss of RPE cell markers or characteristics. Promotion of differentiation may include maintenance or improved retention, or an increase in, or restoration of markers or features of RPE cell differentiation. Differentiation of the cultured RPE cells may therefore be assessed by examining or looking for features or characteristics of native RPE cells e.g. RPE cell markers. Various RPE cell markers are known (RPE "differentiation markers"), by which RPE cells may be characterised, including for example various proteins expressed by RPE cells such as anti-cellular retinaldehyde-binding protein (CRALBP). Other protein RPE differentiation markers include RPE65.

Markers of RPE cell differentiation are discussed for example in Ahmado et al. Invest Ophthalmol Vis Sci. 2011, 52:7148-7159. In addition to the proteins mentioned above, CRALBP and various proteins involved in melanin biosynthesis and storage may be assessed as RPE differentiation markers e.g. retinol dehydrogenase 10 (RDH 10) and/or retinol dehydrogenase 11 (RDH 11). Circumferential actin is a characteristic of native RPE. The absence of expression of various proteins e.g. Keratin-8 or stress fibres may also be used as a marker of RPE cell differentiation.

Other features of native RPE cells may be used to assess RPE cell differentiation, for example, morphological and/or physiological features of the RPE cells. Normal native RPE cell exhibit a characteristic hexagonal (cobblestone) morphology and grow as a single layer or sheet. Native RPE is also polarised, and this polarity is believed to be important in RPE function (see Sonoda et al. 2009, Nat. Protoc. 4(5), 662-673). A polarised and functional RPE monolayer may be characterised by well-defined tight junctions, apical microvilli and/or membrane transport capability, as well as melanocytic pigmentation. Polarisation may be characterised by the expression of polarity markers, including for example the protein Na⁺/K⁺ ATPase which is an apical marker. Expression of tight junctional proteins such as ZO-1 may also be used to assess polarity. The ZO-1 barrier is involved in creating a polarised epithelium and is necessary for maintaining an apical-basal concentration gradient across the RPE. Expression of such a protein and the presence of well developed functional tight junctions may therefore be used as a marker of polarity. Such features can be analysed using methods known in the art, for example as used in the Examples below. Further, tight junctions may be identified by ultrastructural evaluation of the junctional complexes by electron microscopy and/or by the presence of a high transepithelial resistance (TER), which may be assessed by methods known in the art, as described for example in Ahmado (supra).

The present invention may thus be used to promote one or more various aspects of RPE cell differentiation in cultured RPE cells, including in particular the development of an RPE monolayer, RPE cells with tight cell to cell junctions and/or a polarised RPE.

Results reported in Example 1 below show differential gene expression in cultured RPE cells in the presence of sericin, as compared to the absence of sericin. In particular, a number of genes involved in pigmentation and in the visual cycle are up-regulated in the presence of sericin. Thus, the up-regulation of such genes including CRALBP, RPE65, RDH10 for example, may be assessed as a marker of RPE cell differentiation.

Further, gene expression analysis reveals that activation of the NPκB pathway is involved in the promotion of pigmentation and/or differentiation by sericin, although activation of this pathway in itself is not sufficient. Thus, without wishing to be bound by theory, it is proposed that sericin promotes pigmentation and/or differentiation of RPE cells or RPE precursor cells at least in part by activation of the NPκB pathway.

RPE cell morphology and pigmentation (melanogenesis) may be assessed using light microscopy and spectrophotometry. The level of anti-cellular retinaldehyde-binding protein (CRALBP) can also be assessed as a marker of RPE cell differentiation as described in the Examples.

Beneficially, culture of RPE cells in the presence of sericin according to the present invention results in a more pronounced (differentiated) RPE phenotype, but without compromising cell density. Indeed cell density may be improved by using sericin, as compared with growth under other conditions, for example in the presence of serum. Sericin appears to support RPE cell confluence. This supports that sericin may successfully be used as serum-replacement for RPE cells, without decreased cell density. Without wishing to be bound by theory, this beneficial effect on cell density may be related, at least in part, to an effect of improving the viability of the RPE cells.

Cell culture generally requires particular conditions which promote or stimulate cell growth. Generally such growth-permissive or conducive conditions include incubation at elevated temperatures, notably at 37°C, and addition or supply of CO₂. Cells are typically cultured at 37°C in the presence of 95% air and 5% CO₂. Such conditions form preferred embodiments of the invention. More generally the cells may be grown at temperatures from 30 to 37 °C under air together with a supply of CO₂. If desired, low oxygen conditions may be used, for example lower than 20% oxygen. This may be achieved using specific incubators which are known and available in the art.

The cells may be cultured in any convenient or appropriate container or vessel. Any known culture system may be used. The form of the container is not critical, but generally it will be designed or selected to protect the cells from the environment and in particular to avoid or minimise microbial contamination. Suitable containers are known and described in the art, e.g. a dish, well, plate, chamber, flask or bottle etc. The container may or may not be provided with a means for temperature control. The use of a permeable support may facilitate the growth of cells in a polarised state and thus a membrane-based culture system may also be used, for example a Transwell culture system, as used by Sonoda *et al.* (supra).

The RPE is the pigmented cell layer, just outside the neurosensory retina, that nourishes retinal visual cells and is firmly attached to the underlying choroid and overlying retinal visual cells. It is composed of a single layer of hexagonal cells that are densely packed with pigment granules. The term "RPE cells" as used herein includes cells organised as the epithelium layer, e.g. the epithelium tissue, or individual or separated cells, namely cells that are not so organised. Thus, RPE cells or RPE precursor cells may be cultured to produce RPE cells in the form of an RPE cell suspension, or more particularly in the form of a cell suspension comprising RPE cells, or in the form of an intact cell sheet or cell layer (more particularly a cell sheet or cell layer comprising RPE cells). Indeed the cells may be present in any form, whether organised, partially organised, disorganised or not organised.

An "RPE precursor cell" may be any precursor or progenitor cell which can (i.e. which has the capacity or ability to) differentiate into a mature RPE cell, for example a fully differentiated RPE cell. Included as RPE precursor cells are stem cells, including both pluripotent stem cells and more committed stem cells (e.g. totipotent or multipotent stem cells). In particular embryonic stem cells, including human and non-human embryonic stem cells, and induced pluripotent stem cells, including human induced pluripotent stem cells, are included. Thus, non-embryonic stem cells may be used, as may cell lines derived from human or non-human embryonic stem cells. Human embryonic stem cell lines are available which did not involve the destruction of human embryos, and techniques for preparing such cells lines are known. Accordingly, in one embodiment human embryonic stem cells may be used which were derived from human embryos without destroying the embryo. For the preparation of RPE cells for the treatment of human subjects, the use of human precursor cells is preferred.

The cells for culture may be cells as harvested or collected from a donor subject, or they may be cultured cells, e.g. cells cultured from donor cells or tissue or cell lines. The donor subject may be any mammalian subject, including human and non-human mammals. Thus the mammal may be a domestic or livestock animal, a laboratory animal, or indeed any animal of interest. In a preferred embodiment the donor subject is human, and in one embodiment it may be a subject in need of RPE replacement therapy. The invention may be used in the culture of cells for autologous transplant, or in the preparation of cells for allo- or xenotransplantation, e.g. for culture of allograft cells or tissue. Furthermore, the RPE cells include not only primary cells, such as Human Iris Pigment Epithelial Cells (HIPEpiC) (ScienCell Research Laboratory) and their progeny but also RPE cell lines. A number of RPE cell lines are known and include for example the ARPE-19 cell line, available from the ATCC (Rockville, MD), the D407 cell line and h1RPE7 (Sigma-Aldrich).

In a further aspect the present invention also extends to an RPE cell or population of RPE cells cultured according to the present invention, wherein said RPE cell or population of cells is characterised by increased expression of NF-κB1 and NF-κB2, and unaltered expression of tyrosinase, relative to a control RPE cell or population of cells not cultured in sericin. Also provided is such a cell or cell population for use in therapy, as described hereinafter.

Sericin is one of the two major silk proteins. It is a globular protein, constituting about 25-30 % of all silk proteins, and functions to "glue" or hold together the filaments of the fibrous protein fibroin. It occurs in layers enveloping the fibroin filaments and helps in the formation of the silk cocoon. Although sericin has been characterised as consisting of 18 amino acids, having a serine content of 32 %, a total amount of hydroxy amino acids of 45.8 % and the chemical composition C₃₀H₄₀N₁₀O₁, as has been widely reported, the sericin protein may be isolated or obtained in various forms - both molecular weight and secondary structure can vary - depending on the way in which it is prepared (see for example Aramwit et al. 2012, Waste Management & Research, 30(3), 217-224; Padamwar and Pawar, 2004, Journal of Scientific & Industrial Research, 63, 323-329; Terada et al. 2005, Journal of Bioscience and Bioengineering, 100(6), 667-671; and Kato *et al.* 1998 (supra)). Sericin may thus be regarded as a mixture of different proteins with different molecular properties. It exists, or may be prepared or obtained, in a wide range of molecular weights, e.g. from 5 or 10 to over 400 kDa, depending on extraction methods, temperature, pH and processing time. Sericin extracted by different methods can provide different amino acid compositions. Heat or acid extraction give sericin with a molecular weight of from 35-150 kDa, whereas sericin extracted by alkaline solution has a molecular weight of from 15-75 kDa. Sericin extracted by heat, acid and alkaline solution normally shows broad disperse bands on SDS electrophoresis, whereas urea extraction of sericin may produce distinct bands between 10 to >225 kDa. Sericin with a low molecular weight, commonly less than 20 kDa, is soluble in cold water and can be recovered during the early stages of silk production, whereas higher molecular weight sericin is soluble in hot water and can be obtained from the later stages (Aramwit *et al.* 2012 supra).

Thus according to various reports sericin can be classified into different fractions, depending on solubility or method of extraction. For example, as reported in Padamwar and Pawar 2004 (supra) sericin may be classified as sericin A, sericin B, and sericin C, depending on solubility. Sericin A is insoluble in hot water, and contains approximately 17.2 % nitrogen and amino acids such as serine, threonine, glycine, and aspartic acid. On acid hydrolysis sericin B yields the same amino acids as sericin A, but also tryptophan. Sericin C is insoluble in hot water and, on acid hydrolysis it yields proline in addition to the amino acids of sericin B. Various fractions of sericin are also alternatively designated by other parties depending on their dissolution behaviour as sericin A and B, or sericin I, II, III, and IV, or S1, S2, S3, S4, and S5, and as α, β, and γ modifications (see also Padamwar and Pawar 2004 supra).

As described in Terada *et al.* 2005 (supra), different types, or fractions, of sericin with different molecular weights may be obtained depending upon whether or not the extraction method involves or includes hydrolysis. The hydrolysed sericin-S (sericin small) has a molecular weight ranging from 5 to 100 kDa and may be prepared according to the method of Kato 1998 (supra). Sericin-L (sericin large) prepared under non-hydrolysing conditions has a molecular weight ranging from 50 to 200 kDa and may be prepared as described by Terada *et al. 2005* (supra). Other sericin preparations varying in molecular weight, including with different levels of hydrolysis, are also described in Terada *et al.* (supra), namely "fraction L" (20-70 kDa), "fraction M" (10-40 kDa) and "fraction S" (less than 30 kDa) as obtained by gel chromatography of sericin-S and other sericin preparations prepared under various hydrolysis conditions and having different molecular weights as follows: more than 70, 20-70,10-40, 7-25 and less than 10 kDa. Thus varying preparations of sericin protein may be obtained by varying extraction and/or hydrolysis conditions. Procedures and methods for this are known and described in the art.

The term "sericin" as used herein encompasses any of the above forms or types of sericin, including hydrolysed or other fractions. It thus encompasses any fragment or part of the sericin protein that has the required function in RPE cell culture, i.e. facilitates growth, and/or promotes pigmentation and/or differentiation of RPE cells in culture. As well as sericin prepared by extraction from natural sources, the term "sericin" includes also sericin prepared by synthetic means, including by recombinant expression. Thus recombinant sericin peptides are also included. Various recombinant sericin peptides are described in Terada *et al.* 2005 (supra).

In one representative embodiment the sericin may be a hydrolysed sericin preparation (hydrolysed sericin), for example sericin-S or a sericin preparation with a molecular weight up to about 100 kDa, e.g. in the range 20 to 100 or 20 to 70 kDa. Sericin is commercially available and may be obtained from any suitable source, e.g. Sigma-Aldrich (St Louis, MO), or Wako Chemicals USA, Inc. (Richmond, VA). Any commercial preparation of sericin may be used, particularly those sold for the purpose of cell culture.

Sericin may be added to the culture medium for example at 0.01 to 10% (w/v), or any one of 0.01, 0.05, 0.1, 0.2, or 0.5 to any one of 10, 8, 6, 5, 4, 3 or 2%, more particularly 0.1 to 5, 0.1 to 4, 0.1 to 3, 0.1 to 2% (w/v) (i.e. wherein 1% sericin=10mg/mL). In a preferred embodiment sericin is added at 1%(w/v).

The culture medium used to culture the RPE cells according to the present invention may be any medium suitable or appropriate for the culture of mammalian cells, or more particularly for RPE cells. A range of different culture media are known in the art and reported in the literature, and a large number of media are commercially available for this purpose, including media reported for use with RPE cells. Any of these may be used. As noted above, advantageously the medium may be serum-free, and in particular it may be free from any animal-derived or not fully characterised components (e.g. components derived from any living source). Preferably, therefore, the medium may be a chemically-defined medium, that is a medium which contains only specified components, particularly components of known chemical structure. In particular, it may be a synthetic medium, e.g. consisting essentially of synthetically prepared ingredients. In a further preferred embodiment the medium is free from DMSO, preferably serum-free and free from DMSO.

Conveniently the medium is or comprises a minimal medium. A number of minimal media for use in the growth and/or maintenance of mammalian cells are known in the art, and any known or proposed minimal medium may be used. Minimal media have been used for the culture of RPE cells, but conveniently or advantageously they may be supplemented or enriched with further nutritional components or additives. A nutrient-enriched minimal medium may be used. In this context a minimal medium is a basal medium. As noted above, a number of such basal media are known, or can be designed, and contain the minimal ingredients required to maintain, or compatible with maintaining, the cells in a viable state and with allowing them to grow. Basal media can be supplemented with other ingredients or additives according to their desired use, or intended effect.

A minimal medium will generally contain a carbon source or substrate, as a source of energy, amino acids, inorganic salts and vitamins. Thus according to the invention a minimal medium contains a carbon source, a nitrogen source (generally at least one amino acid), at least one inorganic salt and at least one vitamin. Generally it will contain a number of different amino acids, a number of different inorganic salts and a number of vitamins, in other words a multiplicity (i.e. at least 2, 3, 4, 5 or 6) of amino acids, inorganic salts and vitamins.

The carbon source is generally a carbohydrate, e.g. saccharide, generally a sugar, and most preferably a monosaccharide e.g. a hexose, or pyruvate, or a similar or other carboxylic acid, e.g., a 2-keto acid. Typically it will be glucose, and in particular D-glucose (dextrose). In one preferred embodiment of the invention the medium comprises glucose and/or pyruvate, e.g. DMEM comprising glucose and pyruvate. Media may be prepared with varying concentrations of glucose. Typically many commercial media may have glucose in a concentration of about 1 g/L but so-called "high glucose" modifications of various media are available, e.g. having a glucose concentration of 4.5 g/L. Such high glucose media may be used according to the present invention. In one embodiment the medium may have a high level of glucose, for example a concentration in the range of 3 to 6, preferably 4 to 5 g/L, e.g. 4.5 g/L.

The medium may contain all 20 natural (or standard) amino acids or a selection of these, e.g. at least 8, 10, 12 or 13 amino acids. In a representative embodiment the medium may contain the nine so-called essential amino acids (histidine, isoleucine, leucine, lysine, methionine, phenylalanine, threonine, tryptophan, and valine). In another embodiment the medium may contain the following 12 amino acids: arginine, cysteine or cystine, histidine, isoleucine, leucine, lysine, methionine, phenylalanine, threonine, tryptophan, tyrosine, and valine. In a further embodiment the 9 or 12 amino acids may additionally include glutamine and/or a glutamine derivative e.g. the dipeptide L-alanyl-L-glutamine which is a stabilised form of glutamine. Other amino acids may also be added e.g. serine or proline. In further representative embodiments the amino acids listed above (with or without glutamine and/or a glutamine derivative) may additionally contain alanine, asparagine, aspartate, glutamate, glycine, proline and serine. The amino acids may be contained in any form, including salts. Generally they will be L-amino acids. Concentrations of amino acids may vary, and media are commercially available with different concentrations. For example the commercially available Dulbecco's Modified Eagle's Medium (DMEM), which is available in various forms, comprises a four-fold higher concentration of amino acids (and vitamins) as compared with Basal Medium Eagle (BME). The vitamins may comprise choline chloride, partial polyoxometalate calcium (e.g. calcium pantothenate), folic acid, niacinamide (nicotinamide), pyridoxine (e.g. pyridoxal hydrochloride), riboflavin, thiamine, and inositol. The vitamins may be used in any form, e.g. as salts. Other vitamins may also be added e.g. biotin or Vitamin B12.

The inorganic salts may provide essential inorganic ions, and trace elements if desired. For example the inorganic salts may provide a source of Na⁺ and/or K⁺, and Ca²⁺ and/or Mg²⁺ ions. Optionally salts providing Cu²⁺, Zn²⁺, Fe²⁺, Fe³⁺ and/or Co²⁺ ions may be included. In a representative embodiment the salts may include CaCl₂, KCL, MgSO₄, NaCl, and NaH₂PO₄, and optionally NaHCO₃. Many commercially available media for cell culture are prepared using so-called Eagle's salts but media with other salt preparations (e.g. Hank's salts) may also be used, and indeed any suitable or desired salt preparation may be used.

The suitable pH for most cells is 7.2 -7.4. The pH of the medium may accordingly lie in this range, or more generally in the range pH 6.9 to pH 7.5, e.g. pH 7.0 to 7.5 or 7.0 to 7.4. The medium will therefore advantageously have some buffer capacity. Commonly this may be achieved by including bicarbonate salt. Many media employ a NaHCO₃-CO₂ buffer system and this may be included according to the present invention. One or more buffers, or buffer salts, may be added to, or included in the medium. For example, a HEPES/bicarbonate buffer may be used. Other buffer components that may be utilised include NaH₂PO₄, Na₂HPO₄ and β-glycerophosphate. Thus other buffering systems may be used and the medium need not contain bicarbonate. However, in one embodiment the medium contains a bicarbonate salt.

As noted above, any of the known minimal or basal media may be used as the culture medium according to the invention, or as the basis of the medium for use according to the invention. Thus known media may be modified or mixed or combined for use in the present invention. Any of the media known as Minimal Essential Medium (MEM) may be used, in particular any Minimal Essential Medium Eagle or any DMEM, e.g. as available from Sigma-Aldrich. Particular reference may be made to Minimal Essential Medium α-modification (αMEM), which contains non-essential amino acids, sodium pyruvate, and additional vitamins and to DMEM high glucose with pyruvate). MEM Eagle and DMEM including αMEM and high glucose, pyruvate DMEM are available in various forms, and any of these may be used.

Thus, as a representative general listing, the following media may be used: Eagles Minimal Essential Medium, Minimal Essential Medium Eagle with α modification (αMEM), Dulbecco's Modified Eagle's Medium (DMEM), DMEM/F12, IMDM, Medium 199, Medium 109, RPMI 1640, Ham F10, Ham F12, McCoy's 5A.

In a preferred embodiment the medium is selected from DMEM, especially high glucose DMEM and especially high glucose and pyruvate DMEM, and αMEM. In a representative embodiment the αMEM is M4526 from Sigma-Aldrich and the DMEM is 11995 from Thermo Fisher Scientific with high glucose, pyruvate, sodium bicarbonate and L-glutamine.

The media may include or comprise other components or additives such as one or more antibiotics, and/or a pH indicator. Suitable antibiotics include gentamycin, penicillin, streptomycin, doxycycline, and vancomycin. The antibiotic(s) may be present at a concentration of 0.01 to 10% (w/v), or any one of 0.01, 0.05, 0.1. 0.2, or 0.5 to any one of 10, 8, 6, 5, 4, 3 or 2%, more particularly 0.1 to 5, 0.1 to 4, 0.1 to 3, 0.1 to 2% (w/v) (i.e. wherein 1% antibiotic=10mg/mL).

In one embodiment of the invention the medium comprises penicillin and/or streptomycin. For example, in one embodiment the medium is a minimal medium such as αMEM or DMEM, e.g. the minimal medium can be DMEM which comprises penicillin and streptomycin. In a preferred embodiment the DMEM comprises approximately 10.000U penicillin and approximately 10mg streptomycin, preferably at a final concentration of 1%.

A suitable pH indicator is phenol red, but others are known and may be used.

Also included may be one or more other additives or supplements which may improve or enhance RPE cell growth, viability and/or differentiation. Such a further additive may be a hormone.

An example of a hormone which may be used is hydrocortisone or another corticosteroid or analogue of hydrocortisone. Triamcinolone acetonide is a further corticosteroid that could be used. In a preferred embodiment hydrocortisone is added to the medium. The amount of hydrocortisone added to e.g. 500ml of medium may be 1-50µg, e.g. 1, 5, 10, 15, 0, 25, 30, 35, 40, 45 or 50 µg, preferably 10µg. In one preferred embodiment hydrocortisone is added to αMEM.

In another embodiment, the thyroid hormone triiodo-thyronine (also known as T₃) may be added to the medium. The amount of triiodo-thyronine added to 500ml medium may be 0.0030-0.01 µg, e.g. 0.0030, 0.0040, 0.0050, 0.0060, 0.0065, 0.007, 0.008, 0.009 or 0.01 µg, preferably 0.0065µg. In one preferred embodiment the T₃ is added to αMEM Growth supplements may also be added to the medium, for example the N1 medium supplement available from Sigma Aldrich (St. Louis, MO). This growth supplement comprises 50mg/ml transferrin, 5mg/ml insulin, 100mM putrescine, 20nM progesterone, 20nM selenium and 10ng/ml biotin. In a preferred embodiment 1-30ml of N1 growth supplement (100x) may be added to 500ml of medium, e.g. 1, 2, 5, 7, 10, 15, 20, 25 or 30ml, preferably 5ml is added to 500ml medium. In one preferred embodiment the growth supplement is added to αMEM. In another embodiment epidermal growth factor may be added to the medium.

The sulfonic acid taurine (2-aminoethanesulfonic acid) may also be added to the medium. The amount of taurine added to 500ml of medium may be 50-250mg, e.g. 50, 100, 125, 150, 200 or 250mg, preferably 125mg. In one preferred embodiment the taurine is added to αMEM.

Where as discussed above, a minimal medium is used which comprises only essential amino acids, non-essential amino acids such as alanine, arginine, aspartic acid, cysteine, glutamic acid, glutamine, glycine, proline, serine, tyrosine, asparagine, and/or selenocysteine may also be added to the medium. Any one or more of these amino acids may be added to the medium, e.g. 1-12, e.g. 2-10, 4-8 or 5-7 of these amino acids may be added to the medium. By way of example, MEM non-essential amino acid solution from Sigma Aldrich (100x) may be added to the medium, although any suitable non-essential amino acid additive may also be used. 1-30ml of MEM non-essential amino acid solution (100x) may be added to 500ml of medium, e.g. 1, 2, 5, 7, 10, 15, 20, 25 or 30ml, preferably 5ml. In one embodiment the non-essential amino acids are added to αMEM.

Other amino acids such as those discussed herein can be added to the medium. In a preferred embodiment glutamine is added to the medium. For example, 1 to 50ml of a 200mM solution of glutamine may be added to 500ml of medium, e.g. 1, 2, 5, 7, 10, 15, 20, 25 or 30ml, preferably 5ml. In one embodiment the glutamine is added to αMEM. In a further preferred embodiment glutamine is added in combination with penicillin and streptomycin as discussed herein.

The medium may comprise any one or more of the additives discussed above, i.e. any combinations of these additives may be present in the medium for use according to the invention.

In a preferred embodiment, the medium is DMEM (high glucose, pyruvate) comprising 1% sericin, and penicillin and streptomycin as discussed herein, preferably at the preferred concentrations discussed herein.

In an alternative preferred embodiment the medium is αMEM (e.g. M 4526) comprising 1% sericin, N1 growth supplement, taurine, triiodo-thyronine, non-essential amino acids, glutamine-penicillin-streptomycin and hydrocortisone as discussed herein, preferably at the preferred concentrations discussed herein.

A further example of an additive which may be added to any of the above-discussed media is an antioxidant. A number of antioxidants are known, and a number of compounds or substances have been reported to have antioxidant activity or properties. Any such compound may additionally be used according to the present invention. Particular reference may be made to DADLE ([D-Ala², D-leu⁵]-enkephalin), a stable analogue of endogenous δ opoid enkephalin. DADLE or other enkephalins or enkephalin analogues may further added to the medium.

The invention may be used to culture RPE cells for a suitable period of time, for example to generate sufficient RPE cells for transplantation purposes. The time used or taken may depend upon the cells which are cultured, for example whether they are RPE cells or RPE precursor cells e.g. stem cells. Thus, the RPE cells may be cultured for shorter periods, for example up to 30, 22, or 15 days, or longer periods, for example up to 8, 10, or 12 weeks or longer, e.g. up to 3 months. For example, the cells may be cultured for up to 22, 21, 20, 19, 18, 17, 16, 15, 14, 13, 12, 11 or 10 days, or for short periods of about a week, or a week or so, e.g. up to 7, 10 or 14 days. Alternatively, the cells may be cultured for longer periods, e.g. up to 90, 85, 80, 75, 70, 65, 60, 55, 50, 45, 40, 35, 30, 25, 24 or 23, days, e.g. for up to 3 months, 2 months, or 12, 11, 10, 9, 8, 7, 6, 5, 4, 3 or 2 weeks.

Culture may take place using standard procedures and culture vessels as discussed above. Typically culture medium may be changed every 2, 3 or 4 days e.g. every 2 or 3 days.

The invention may be used whenever and wherever the culture of RPE cells may be helpful or desired. Although the field of cells/tissue for transplantation is a particular area of utility, the invention is not limited to this and may be used in any context, including other therapeutic contexts, production of cell products, laboratory use, research etc.

Thus, the present invention may be used to culture RPE cells for use in therapy, for example the treatment or prevention of an ophthalmological condition, notably a condition involving the RPE, such as AMD. That is, RPE cells cultured according to the present invention may be used in the treatment or prevention of an ophthalmological condition, especially a condition involving the RPE, such as AMD.

As such, in one embodiment the present invention provides RPE cells according to the invention for use in therapy. In a preferred embodiment the provided RPE cells are for use in the treatment or prevention of an ophthalmological condition, preferably a condition involving the RPE, more preferably AMD.

An "ophthalmological condition" is any disorder, condition or disease involving the eye. Generally speaking cultured RPE cells obtained according to the present invention may be used to treat or prevent an ophthalmological condition involving the RPE. As discussed herein, one example of such a condition is age-related macular degeneration, and the present invention can therefore be used to culture RPE cells which can be used therapeutically in the treatment of age-related macular degeneration, or other ophthalmological conditions involving RPE cells. Other examples of ophthalmological conditions involving RPE which may be treated or prevented by the present invention include Retinitis pigmentosa and Stargardt's disease. In such a case the cells may be administered locally, i.e. directly to the appropriate region of the eye i.e. to the interior of the eye, or to the retina or RPE e.g. by injection into the eye. Alternatively expressed, the RPE cells cultured according to the present invention, i.e. in a medium comprising sericin, may be used in the treatment of any condition responsive to RPE cell replacement therapy, e.g. any condition in which an RPE transplant would be indicated or in which augmentation of RPE cell survival would assist or be of benefit.

Alternatively put, RPE cells cultured according to the present invention may be used in the manufacture of a medicament for treating or preventing an ophthalmological condition, preferably a condition involving the RPE, more preferably AMD.

Thus, the RPE cells cultured according to the invention may be provided in the form of a pharmaceutical composition, i.e. comprising one or more pharmaceutically acceptable diluents, carriers or excipients.

These compositions may be formulated in any convenient manner according to techniques and procedures known in the pharmaceutical art, e.g. using one or more pharmaceutically acceptable diluents, carriers or excipients. "Pharmaceutically acceptable" as referred to herein refers to ingredients that are compatible with other ingredients of the compositions (or products) as well as physiologically acceptable to the recipient. The nature of the composition and carriers or excipient materials, dosages etc. may be selected in routine manner according to choice and the desired route of administration, purpose of treatment etc. Dosages may likewise be determined in routine manner and may depend upon the nature of the molecule (or components of the composition), purpose of treatment, age of patient, mode of administration etc.

As defined herein "treatment" refers to reducing, alleviating or eliminating one or more symptoms of the condition which is being treated, relative to the symptoms prior to treatment.

"Prevention" refers to delaying or preventing the onset of the symptoms of the condition. Prevention may be absolute (such that no condition occurs) or may be effective only in some individuals or for a limited amount of time.

For *in vivo* administration of the RPE cells, any suitable mode of administration of the cell or cell population which is common or standard in the art may be used, e.g. injection or local administration by an appropriate route. Preferably 1x10⁴ to 1x10⁸ cells are administered per kg of subject (e.g. 1.4x10⁴ to 2.8x10⁶ per kg in human). In one embodiment a lower dose of cells may be given for example 4x10³, 3 x 10³, 2x10³ or 1x10³ cells may be administered per kg of subject. Thus, for example, in a human, a dose of 0.1-20x10⁷ cells may be administered in a dose, i.e. per dose. The dose can be repeated at later times if necessary.

The invention will now be described in more detail in the Examples below with reference to the following drawings in which:

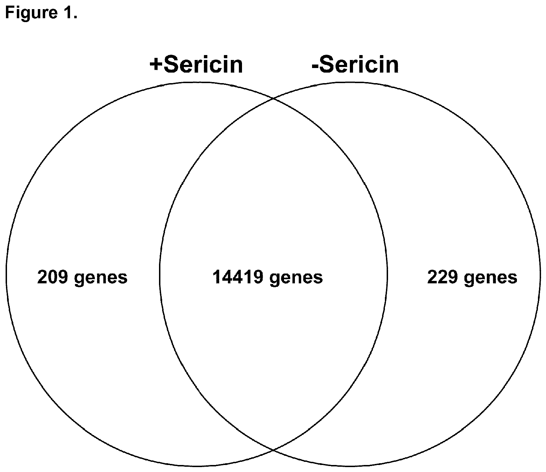

Figure 1 shows the results of a study in which the effect of sericin on gene expression was analysed by RNA microarray. HRPE cells were cultured for twelve days in DMEM with or without 1% sericin and were. The Venn diagram illustrates the number of transcripts with changed levels (fold change > 1.5, nominal P < 0.05). 209 and 229 transcripts were increased or reduced, respectively, while 14419 transcripts were unchanged. Transcripts with maximal log₂ transformed signal values less than 5 were removed to filter for low and non-expressed genes.

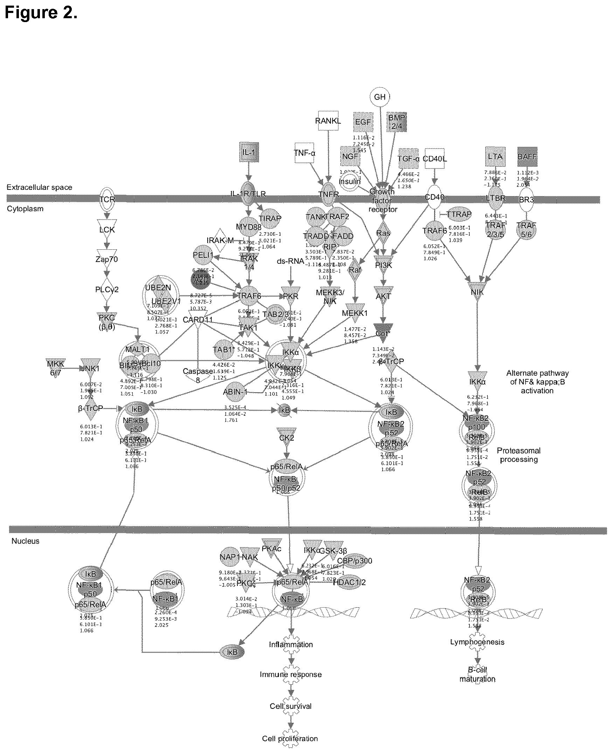

Figure 2 shows the results of a study analysing transcripts that changed upon addition of sericin. The NF-κB complex was among the most highly activated (z-score: 4.597) upstream regulators. IPA revealed up-regulation of transcripts belonging to both the main and alternate NF-κB pathway. Activation of the NF-κB pathway was indicated by up-regulation of its components, including A20, Cot and NF-κB2 p100.

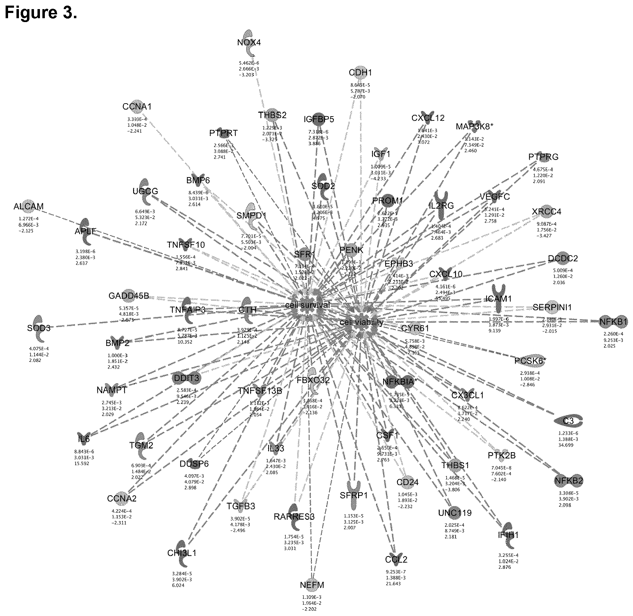

Figure 3 shows the results of pathway analyses which predict the downstream effects of culturing of HRPE cells with or without sericin. Ingenuity Pathway Analysis predicted a downstream effect compatible with increased viability and survival of human retinal pigment epithelial cells cultured in DMEM with 1% sericin compared to cells cultured without sericin. In the cell viability category (right center), 37 of 57 genes had an expression direction consistent with increased cell viability, yielding a Z-score of 2.8. In the cell survival category (left center), 38 of 61 genes had an expression direction consistent with increased cell survival, yielding a Z-score of 2.7. The upper two numbers below each symbols indicate P-values for cell survival or cell viability, respectively, while the lower number indicate fold change.

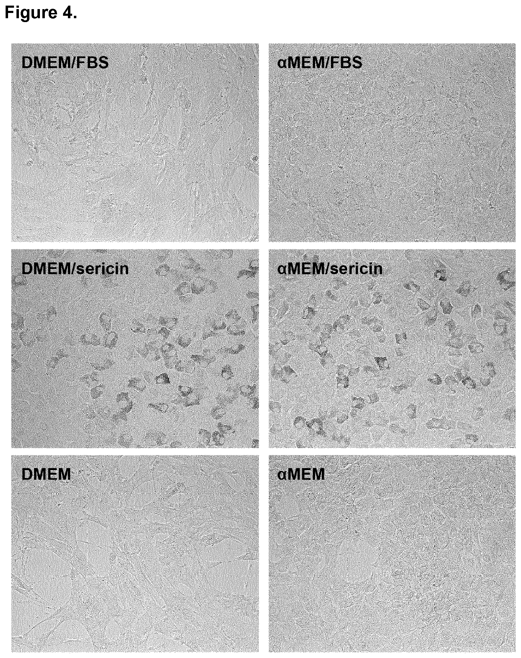

Figure 4 shows photomicrographs showing human retinal pigment epithelial cells cultured for 14 days with or without 1% sericin using two different basal media. Magnification: 200x. Photomicrographs are representative of eight samples.

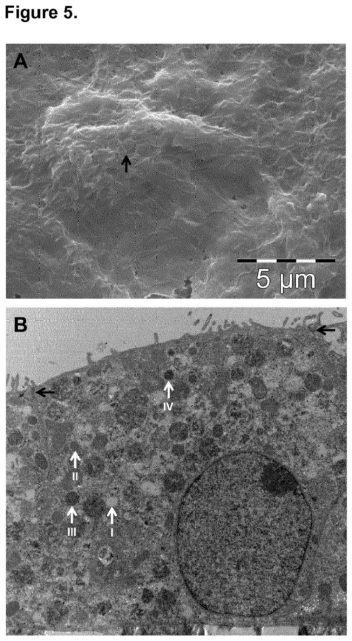

Figure 5 shows the ultrastrucure of HRPE cells cultured with sericin. Human retinal pigment epithelial HRPE cells were cultured in DMEM with 1% sericin for twelve days and then analysed by electron microscopy. (A) Scanning electron microscopy image showing the apical surface of a confluent layer of hexagonal HRPE cells with extensive microvilli (arrow). (B) Transmission electron microscopy image showing a polarized HRPE cell with apical tight junctions (black arrows), a basal nuclei and numerous melanosomes between stage I (non-melanized) and stage IV (melanized).

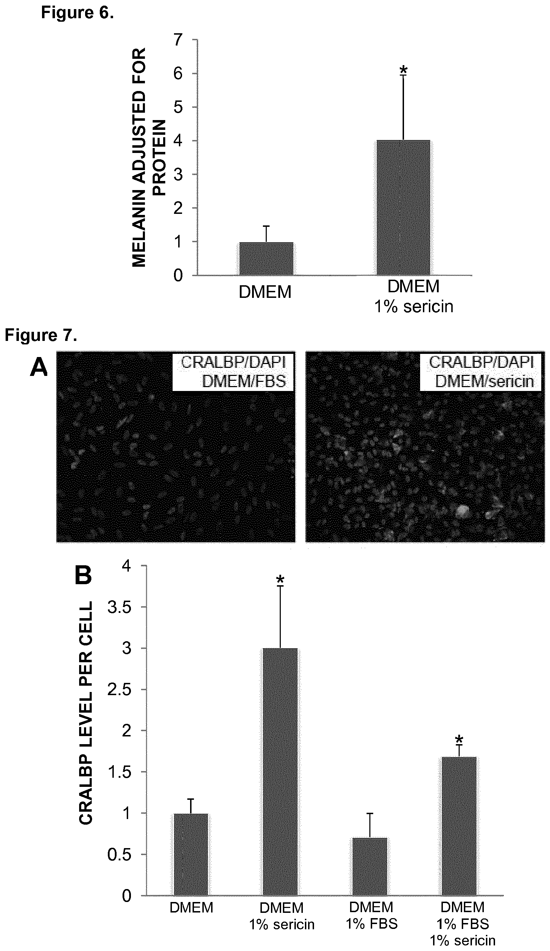

Figure 6 shows the results of a study in which melanin content was measured by spectrophotometry. The bars show total protein adjusted relative levels of melanin after culturing HRPE cells for seven days with or without sericin as described. * P<0.05
Figure 7 shows the results of a study in which the expression of the cellular retinaldehyde-binding protein (CRALBP) was measured by quantitative immunofluorescence in human retinal pigment epithelial cells cultured for 14 days in DMEM with or without 1% sericin and/or fetal bovine serum (FBS). (A) Photomicrographs show that CRALBP expression (red) was lowest in the DMEM/FBS group and highest in the DMEM/sericin group. Cell nuclei were counterstained with DAPI (blue). Magnification: 200x. Photomicrographs are representative of four to eight samples. (B) Bar chart showing CRALBP expression in fold change relative to the DMEM alone group. N = 4 to 8. Error bars: standard deviation. * P < 0.01 compared to all other groups.

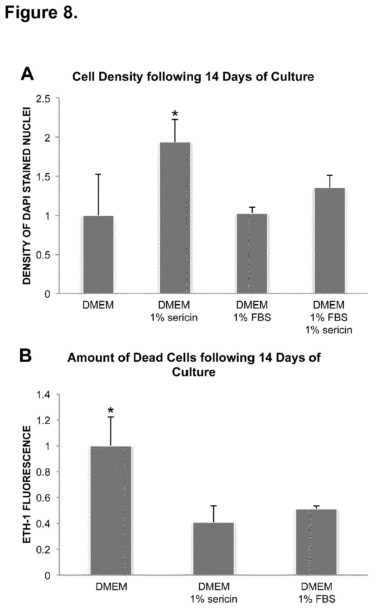

Figure 8 shows the results of a study in which cell density and cell survival were measured by quantitative fluorescence in human retinal pigment epithelial cells cultured in DMEM with or without sericin or fetal bovine serum (FBS) for 14 days. (A) Bar chart showing cell density in fold change relative to the DMEM-group. Cell density was measured by counting DAPI-stained nuclei using ImageJ. N = 8. Error bars: standard deviation. * P<0.001 compared to DMEM and DMEM with FBS; P=0.037 compared to DMEM with sericin and FBS. (B) Bar chart showing amount of dead cells in fold change relative to the DMEM-group. Cell death was measure by ethidium homodimer-1 (ETH-1) uptake by dead cells using a microplate reader. * P<0.001 compared to other two groups.

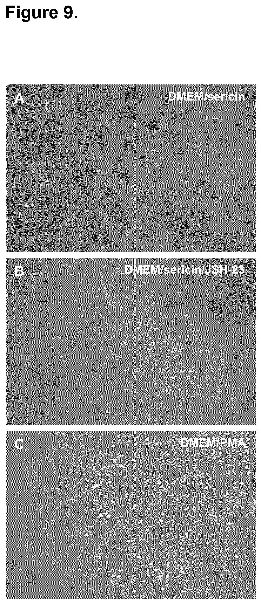

Figure 9 shows the results of a study in which NF-κB signalling was stimulated or inhibited in human retinal pigment epithelial cells. The NF-κB activator inhibitor 4-methyl-N1-(3-phenylpropyl)-1,2-benzenediamine (JSH-23) and the NF-κB agonist phorbol-12-myristate-13-acetate (PMA) were used to assess whether activation of the NF-κB pathway is necessary and sufficient for sericin-induced pigmentation of human retinal pigment epithelial (HRPE) cells following seven days of culture. (A) Photomicrograph showing pigment-containing HRPE cells following culture in DMEM with sericin. Magnification: x20. (B) Photomicrograph showing absence of pigment in HRPE cells following culture in DMEM with sericin and JSH-23. Magnification: x20. (C) Photomicrograph showing scarce pigment in HRPE cells following culture in DMEM with PMA. Magnification: x20.

### Examples

### Example 1

This study was carried out to determine whether a medium containing sericin is suitable for culture of RPE cells.

### Materials and Methods

Normal HRPE and complete epithelial cell medium (EpiCM) were obtained from ScienCell Research Laboratories (San Diego, CA). Dulbecco's Modified Eagle's Medium (high glucose, with pyruvate; hereafter named DMEM), Minimal Essential Medium (a-modification; MEM-α), heat-inactivated fetal bovine serum (FBS), N1 growth supplement, taurine, triiodo-thyronine, non-essential amino acids, glutamine-penicillin-streptomycin, hydrocortisone, propidium iodide (PI) and 4',6-diamidino-2-phenylindole (DAPI) and 4-methyl-N1-(3-phenylpropyl)-1,2-benzenediamine (JSH-23) were obtained from Sigma Aldrich (St Louis, MO). Nunclon Δ surface 96-well plates, pipettes and other routine plastics came from VWR International (West Chester, PA). Mouse monoclonal anti-cellular retinaldehyde-binding protein (CRALBP; clone B2) was from Abcam (Cambridge, UK). Ethidium homodimer-1 (ETH-1) was from Invitrogen. Secondary Cy3-conjugated anti-mouse antibody was from Abcam. Ethidium homodimer 1 (EH-1) was obtained from Invitrogen (Carlsbad, CA). Soluene®-350 was obtained from PerkinElmer (Waltham, MA). Pierce BCA Protein Assay Kit was obtained from Bio-Rad (Hercules, CA).

### Cell Culture

Third passage HRPE were seeded (7000 cells/cm²) in complete EpiCM on Nunclon Δ surface 96-well plates and cultured under routine conditions of 95% air and 5% CO₂ at 37°C. After two days, EpiCM was replaced with either: 1) DMEM with 1% FBS; 2) DMEM with 1% sericin; 3) DMEM without FBS or sericin; 4) MEM-α with 1% FBS; 5) MEM-α with 1% sericin; or 6) MEM-α without FBS or sericin. All culture media based on DMEM were supplied with 10.000 U penicillin and 10 mg streptymocin at a final concentration of 1%. The MEM-α-based media were added taurine, triiodo-thyronine, non-essential amino acids, glutamine-penicillin-streptomycin, hydrocortisone, and N1 medium supplement, as described in Sonoda *et al.* (supra). The culture medium was changed every two to three days, and the HRPE were maintained in culture for a total of 14 days.

### RNA Extraction and Microarray Hybridization

HRPE cells were cultured for 12 days and washed with PBS and lysed with QIAzol Lysis Reagent. The lysate was transferred to a microcentrifuge vial. Total RNA was then purified according to the manufacturer's protocol, and 100 ng of total RNA was processed with a GeneChip HT One-Cycle cDNA Synthesis Kit and a GeneChip HT IVT Labeling Kit (Affymetrix, Santa Clara, CA). Labeled and fragmented single stranded cDNAs were hybridized to the GeneChip Human Gene 1.0 ST Arrays (28,869 transcripts) (Affymetrix). Thereafter, the arrays were rinsed and stained using a FS-450 fluidics station (Affymetrix). Signal intensities were measured with a Hewlett Packard Gene Array Scanner 3000 7G (Hewlett Packard, Palo Alto, CA), and the scanned images were processed by the Affymetrix GeneChip Command Console (AGCC).The CEL files were imported into Partek Genomics Suite software (Partek, Inc. MO, USA). Robust microarray analysis (RMA) was applied for normalization. Gene transcripts with a maximal signal values less than 32 across all arrays were removed to filter for low and non-expressed genes, reducing the number of gene transcripts to 23190. Differentially expressed genes between groups were identified using one-way ANOVA analysis in Partek Genomics Suite Software. Clustering analysis was made using the same name module in a Partek Genomics Suite Software. Gene transcripts with maximal signal values of less than 5 were removed to filter for low and non-expressed genes, resulting in 14419 gene transcripts. For expression comparisons of different groups, profiles were compared using a 1-way ANOVA model. Data were presented as fold changes (FC) and P-values.

### Microarray Data Analysis

Upstream analysis, pathway analysis and downstream predictions were performed using Ingenuity Pathways Analysis (IPA) (www.ingenuity.com).

### Verification of Affymetrix data by RT-PCR

The differential gene expression data were validated for selected transcripts using TaqMan® Gene Expression Assays and the Applied Biosystems ®ViiA™ 7 Real - Time PCR system (Applied Biosystem, Life technologies, Carlsbad, CA) (Table 1). Affymetrix analysis identified MAPRE1 and POLR5H as ideal for endogenous controls due to their stable expression across samples and groups. Briefly, 200 ng totalRNA were reverse transcribed using qScript™ cDNA Super Mix (Quanta Biosciences, Gaithersburg, MD) following the manufacturer's instructions. After completion of cDNA synthesis 1/10 of the first strand reaction were used for PCR amplification. 9 *µ*l of cDNA (diluted in H₂O), 1 *µ*l of selected primer/probes TaqMan® Gene Expression Assays (Life Technologies) and 10 *µ*l TaqMan® Universal Master Mix (Life Technologies) following the manufacturer's instructions. Normalized ΔCt values were calculated by subtracting the average Ct of the two endogenous controls from the Ct of the reference gene. ΔΔCt values were then calculated by subtracting normalized ΔCt values of the group containing cells cultured in DMEM with 1% sericin from the control group, which included cells cultured in DMEM without sericin. P-values were calculated using Student's t-Test in Microsoft Excel using delta Ct values. Normalized target gene expression levels (FC) were calculated using the formula: 2^{(-ΔΔCt)}.

### Light Microscopy

Cell morphology and presence of pigment was assessed after 14 days of culture by light microscopy at 200x magnification.

### Transmission Electron Microscopy

HRPE cells cultured for 12 days in DMEM with 1 % sericin were processed for transmission electronmicroscopy (TEM) analysis. In brief, ultrathin sections (60-70 nm thick) were cut on a Leica Ultracut Ultramicrotome (Leica, Wetzlar, Germany) and examined using a CM120 transmission electron microscope (Philips, Amsterdam, the Netherlands).

### Scanning Electron Microscopy

HRPE cells cultured on glass coverslips in DMEM with 1 % sericin for 12 days were used for scanning electron microscopy (SEM). Glutaraldehyde-fixed samples (n=3) were dehydrated in increasing ethanol concentrations and then dried according to the critical point method (Polaron E3100 Critical Point Drier, Polaron Equipment Ltd., Watford, UK) with CO₂ as the transitional fluid. The specimens were attached to carbon stubs and coated with a 30nm thick layer of platinum in a Polaron E5100 sputter coater before being photographed with an XL30 ESEM electron microscope (Philips, Amsterdam, The Netherlands).

### Melanin Measurement by Spectrophotometry

Intracellular melanin was quantified in HRPE cultured in DMEM with and without 1% sericin for one week. Seven-day cultures were rinsed with PBS, and then resuspended in 200 *µ*L of RIPA lysis buffer consisting of 25mM Tris-HCL [pH 7.6], 150 mM NaCl, 1% NP-40, 1% sodium deoxycholate and 0.1% sodium dodecyl sulfate dissolved in H₂O. Part of the cell lysates were then incubated with Pierce BCA protein assay kit for 30 minutes at 37°C, and cooled to room temperature before measuring the absorbance at 562 nm using a microplate reader (VERSAmax, Molecular Devices, Sunnyvale, CA). Soluene®-350 was thereafter added to the remaining cell lysate (9:1). The cell lysate was subsequently incubated for 60 minutes at 80°C, before being centrifuged for 10 minutes at 8600 g. Solubilized melanin was measured at 490 nm on the microplate reader, and the concentration was adjusted by multiplication with protein levels.

### Quantitative Immunofluorescence

Following 14 days of culture the cells were fixed in 100% methanol for 15 minutes and then washed three times with fresh PBS. Fixed cells were incubated for 45 minutes at room temperature in a blocking buffer consisting of 10% goat serum, 1% BSA, 0.1% Triton X-100, 0.05% Tween-20, 0.05% sodium azide in PBS. Cells were then incubated overnight at 4°C with the following antibody diluted in blocking buffer: anti-CRALBP (1:100), which targets a functional protein in the visual cycle and a marker for differentiated HRPE. The Cy3-conjugated secondary antibody, diluted in 0.2% PBST with 1% BSA, were incubated for one hour at room temperature. Negative control consisted of replacing the primary antibody with PBS. The cultures were thereafter rinsed three times in PBS and incubated with 1*µ*Lg/mL DAPI in PBS to stain cell nuclei before a final wash with PBS.

Photomicrographs of the cultures were captured at 200x magnification using a Nikon Eclipse Ti fluorescence microscope with a DS-Qi1 black-and-white camera. The exposure length and gain was maintained at a constant level for all samples, and the fluorescence intensities of the Cy3 fluorochromes, which were conjugated to the secondary antibodies, were within the dynamic range of the camera. Phenotype was quantified using ImageJ (National Institutes of Health, Bethesda, MD) as described in e.g. Eidet et al. (Diagnostic Pathology, 2014. 9:92) with some modifications. In brief, mean fluorescence per cell was measured by enlarging regions of interest (ROI) created around the DAPI-stained nuclei to enclose the CRALBP-expressing cytosol. By using this method, we were able to normalize for differences in cell density in each photomicrograph.

### Quantification of Cell Density

After 14 days in culture, the HRPE (n=8) were fixed with methanol, as described above. The cultures were then rinsed three times in PBS and incubated with 1 *µ*g/mL DAPI in PBS to stain cell nuclei before a final wash with PBS. Photomicrographs of the cultures were captured at 200x magnification with identical exposure length and gain. ImageJ was used to convert 16-bit images to binary images. The "Analyze particles" function in ImageJ was then used to automatically count cell nuclei per image.

### Quantification of Cell Death

The amount of dead cells in the cultures after two weeks was quantified by incubating the samples with EH-1 for 30 minutes at 37°C. Ethidium homodimer-1 stains nuclei of dead cells red and its fluorescence was quantified by the microplate fluorometer with the excitation/emission filter pair 530/620. Background fluorescence, measured in wells incubated with EH-1-reagent, but without cells, was subtracted from all values before calculating mean fluorescence for the groups.

### Statistical Analysis

One-way ANOVA with Tukey's post hoc pair-wise comparisons (SPSS ver. 21.0) was used to compare the groups. Data were expressed as mean ± standard deviation, and values were considered significant if P<0.05.

### Results

### Microarray Analysis of HRPE Cultured with or without Sericin

*Global Perspective.* Gene expression differed considerably between the two culture groups, with a total of 438 significantly differentially regulated genes (fold change > 1.5; P < 0.05). In the sericin-supplemented group, 229 genes were down-regulated and 209 genes were up-regulated compared to the control (Figure 1).

*Upstream analysis.* The top upstream regulators were tumor necrosis factor (TNF) (z-score: 5.008), interferon-y (IFN-γ) (z-score: 4.623), the NF-κB complex (z-score: 4.597) and interleukin-1β(IL1β) (z-score: 4.567). Pathway analysis of NF-κB revealed up-regulation of transcripts belonging to both the main and alternate NF-κB pathway (Figure 2). Activation of the NF-κB pathway was indicated by up-regulation of several of its members, including A20, Cot and NF-κB2 p100 (Table 2).

*Substantially Regulated Genes.* The C-X-C motif chemokine 10 (CXCL10, also known as IP-10; interferon gamma-induced protein 10) was the most up-regulated gene in the dataset, with a 55.5-fold up-regulation in the sericin-supplemented group compared to the control (Table 3). Complement component 3 (C3) was the second most up-regulated gene in the sericin group, with a 34.7-fold up-regulation. The chemokine (C-C motif) ligand 2 (CCL2) was up-regulated 21.6-fold in the sericin-supplemented group (Table 3).

*Downstream Effects.* The results obtained from the Ingenuity Pathway Analysis (IPA) predicted a downstream effect compatible with increased cell viability and survival in the sericin-supplemented group (Figure 3). In the cell viability category, 37 of 57 genes had an expression direction consistent with increased cell viability, yielding a Z-score of 2.8. In the cell survival category, 38 of 61 genes had an expression direction consistent with increased cell survival, yielding a Z-score of 2.7.

### RPE Related Gene Transcripts

Pigmentation. The (IPA) detected 12 differentially expressed genes related to cell pigmentation (Table 4). Eleven of the genes were up-regulated and one was down-regulated in HRPE cells cultured in DMEM with sericin compared to cells cultured in DMEM without sericin.

Visual Cycle. Several visual cycle genes were significantly up-regulated in HRPE cells cultured in DMEM with sericin compared to that of cells cultured in DMEM without sericin, while no visual cycle genes were significantly down-regulated (Table 5). Compared to cells cultured in DMEM without sericin, the cells that were cultured in DMEM with sericin displayed a 2.8-fold up-regulation of RPE65, a key isomerase of the visual cycle and an essential marker of RPE cell differentiation, and a 1.4-fold up-regulation of cellular retinaldehyde binding protein 1 (RLBP1; also known as CRALBP), which positions retinol for enzymatic turnover and thereby accelerates the process. Both retinol dehydrogenase 10 (RDH10) and retinol dehydrogenase 11 (RDH11) play complementary roles as 11-cis-retinol dehydrogenases in the visual cycle, and were up-regulated 1.9 and 1.6-fold, respectively, in cells cultured in DMEM with sericin compared to cells cultured in DMEM without sericin.

### RT-PCR Validation

To validate the microarray results, NFKBIA, RPE65, CSF1, NFKBIZ and RGR transcripts were quantified by RT-PCR in HRPE cells cultured for twelve days in DMEM with or without 1% sericin. In Table 6, ΔΔCt values are transformed to fold change. All six transcripts were up-regulated in the Affymetrix and RT-PCR experiments (Table 6).

### Microstructure following HRPE Culture with or without Sericin

Human retinal pigment epithelial cells were cultured with or without 1% sericin in two different basal media with or without FBS for 14 days before being assessed with light microscope for morphology and pigmentation (N = 8). Widespread pigmentation was only seen in cells cultured with 1% sericin, irrespective of the basal medium used (MEM-α or DMEM) (Figure 1). Pigmented cells were rarely found in the groups that were cultivated without sericin. Typical cobblestone morphology with essentially hexagonal cells was seen in the samples cultured in DMEM with sericin, MEM-α with sericin, MEM-α with FBS, and MEM-α alone (Figure 4). Cell confluence was superior in DMEM with 1% sericin compared to DMEM alone and DMEM with 1% FBS. Hence, these results show that after 14 days in culture, only cells cultured in media supplied with sericin become pigmented and sericin appears to support HRPE cell confluence.

### Ultrastructure following HRPE Culture with or without Sericin

Scanning electron microscopy of HRPE cultured for twelve days in DMEM with 1% sericin appeared tightly adjoined, hexagonal and had apical microvilli (Figure 5A), hence demonstrating a differentiated ultrastructure. Transmission electron microscopy of HRPE cultured for twelve days in DMEM with 1% sericin contained melanosomes of all four stages following seven days of culture (Figure 5B), thereby supporting the validity of the light microscopy experiments.

### Melanin Quantification following HRPE Culture with or without Sericin

As the pigment melanin absorbs light at a specific wave length, measurement of pigment quantity is commonly performed by spectrophotometry. Following seven-day culture, spectrophotometry showed increased absorption at 562 nm in cells cultured in DMEM with 1% sericin (4.0 fold ± 0.9; P=0.008) compared to cells cultured in DMEM without sericin (Figure 6). Thus, the results further supports increased pigmentation in sericin-cultured cells.

### Level of the CRALBP Protein following HRPE Culture with or without Sericin

To verify the presence and to assess the quantity of RPE-related proteins quantitative immunofluorescence was used to analyze the mean level of CRALBP, which is a protein integral to the visual cycle and associated with differentiated RPE. Following 14 days of culture, CRALBP was most abundant in cells cultured in DMEM with sericin, and present to a significantly higher degree than in cells cultured in DMEM alone, DMEM with FBS or DMEM with FBS and sericin (P < 0.01) (Figure 7). Retinal pigment epithelial cells cultured in DMEM with sericin and FBS also demonstrated a significantly higher expression of CRALBP compared to the DMEM alone and DMEM with FBS groups (*P <* 0.01). Thus, quantitative immunofluorescence confirmed increased levels of CRALBP, as indicated by the microarray results.

### HRPE Cell Density and Cell Death following Serum Starvation

As the microarray results predicted increased viability of culturing cells in DMEM with sericin compared to DMEM without sericin, cell density and cell death after 14 days of cultivation with or without sericin was quantified by counting DAPI-stained cell nuclei and by microplate fluorometer measurements of EH-1, the latter which is indicative of dead cells. DMEM with sericin yielded the highest cell density and significantly higher density than DMEM alone (P<0.001), DMEM with FBS (P<0.001) and DMEM with sericin and FBS (P=0.037) (Figure 8).

Cultured HRPE were assayed with EH-1 to quantify the number of dead cells following 14 days of culture (N=8). The highest number of dead cells was obtained when using DMEM alone (Figure 8B). Cells cultured in this medium demonstrated a significantly higher amount of dead cells than when using DMEM with sericin or DMEM with FBS (P<0.001). Thus, the cell density and cell death results supported the microarray prediction of increased viability when culturing HRPE in DMEM with sericin.

### NF-κB Pathway and Melanization of Cultured HRPE Cells

The NF-κB-inhibitor JSH-23 specifically inhibits nuclear translocation and activation of NF-κB. The addition of JSH-23 to a culture medium consisting of DMEM with 1% sericin completely prevented development of pigmented cells visible by light microscopy following seven days of culture, as opposed to control cells cultured in DMEM with sericin, but without JSH-23 (Figure 9A and B). However, stimulation of the NF-κB pathway by adding PMA, a NF-κB agonist, to a culture medium consisting of DMEM without sericin did not induce melanization (Figure 9C). Thus, NF-κB activation appears to be necessary, but not sufficient, for sericin-induced melanization of HRPE.

### Discussion

In the current study, sericin induced pigmentation of cultured HRPE by NF-κB pathway activation while still preserving cell viability and improving RPE differentiation, as indicated by pathway analysis of Affymetrix microarray, micro and ultra-structural studies, spectrophotometry, quantitative immunofluorescence and viability assays.

The NF-κB pathway was, alongside TNF, IFN-γ and IL1β, one of the top upstream sericin-induced regulators in this study. The NF-κB pathway is involved in multiple cellular processes, including inflammation and immunity. NF-κB is also part of the TNF pathway and is regulated by IL1β. A link between inflammatory cytokines, including IL1β and TNF-α, and induction of pigmentation in chick RPE cells has been reported. Interestingly, IFN-γ activation has been related to hypopigmentation in skin melanocytes. In our study, the addition of the NF-κB activator inhibitor JSH-23 prevented pigmentation in sericin-cultured cells and thereby confirmed the role of NF-κB in pigmentation of HRPE, whereas the relatively scarce pigmentation achieved with the NF-κB agonist PMA alone suggested that sericin promotes pigmentation, albeit not exclusively, by activating the NF-κB pathway.

IPA identified several genes that are potentially linked to sericin-induced pigmentation, including ABCA4, PROM1, C10orf11, SLC24A5, TGF2 and IRF1, which were all up-regulated by sericin. Of these genes, mutations in ABCA4 have been related to Stargardt disease and retinitis pigmentosa (RP), with associated pigment disturbances. PROM1 is also related to maculopathy, as mutations in this gene may cause macular degeneration, including dominant bull's eye maculopathy. Mutations in C10orf11 have been shown to decrease pigmentation of melanocytes and lead to human albinism. Interestingly, down-regulation of SLC24A5 causes reduced melanin content in chick RPE. Furthermore, SLC24A5 has also been related to melanin content in skin melanocytes and the gene product of SLC24A5 localizes to intracellular membranes, including melanosomes. Inhibition of TGF2 has been reported to suppress melanogenesis in human melanoma cells. Surprisingly, up-regulation of IRF1 has been associated with hypo-pigmentation in skin melanocytes. Thus, of all the genes identified by IPA as being associated with sericin-induced pigmentation in this study, C10orf11, SLC24A5 and TGF2 appear to be the most promising.

To our knowledge there are no studies on the effects of sericin on RPE melanogenesis for direct comparison to our study. However, previous reports have demonstrated that sericin inhibits tyrosinase, which is the main rate-limiting melanogenesis enzyme, thus the induction of pigmentation by sericin in our study is unexpected. Tyrosinase catalyses the formation of dihydroxyphenylalanine (L-DOPA) from L-tyrosine. L-DOPA is subsequently converted to melanin, aided by tyrosinase-related proteins 1 and 2 (TRP-1 and TRP-2). Tyrosinase is inhibited by acidic conditions, including those resulting from high metabolic activity. The microarray data did not reveal any significant effect of sericin on the tyrosinase transcript TYR, or on TRP-1 and TRP-2, thereby suggesting that sericin's effect on pigmentation is unrelated to regulation of these genes. The production of melanin is a complex process, however, involving several steps where sericin could have a stimulating role that fully compensates for its acclaimed tyrosinase-inhibiting effect.

Pigmentation was almost exclusively seen in cells that had been cultured in sericin-supplemented basal media (either MEM-α or DMEM). Hexagonal cobblestone morphology was also achieved when using either basal media supplemented with sericin. After culturing the cell line ARPE-19 for 98 days in DMEM with FBS, Ahmado and co-workers demonstrated pigmented and hexagonal cells Ahmado *et al.* 2011 (supra). To our knowledge, this medium has not been used for normal RPE. The MEM-α-based medium, however, has been shown to induce pigmentation in normal HRPE cells in study by Sonoda *et al.* 2009 (supra). In contrast to our study, the HRPE in their study was pigmented upon start of culture and, after initial depigmentation, became repigmented after 14 days. Both MEM-α and DMEM supplemented with FBS are known to induce differentiation of RPE cells in prolonged culture (>3 weeks /months). Our culture time of seven to 14 days may, therefore, have been too short time for widespread melanogenesis to occur in these media.

In RPE, pigment can be seen within vesicles called melanosomes, which can be divided into four stages of development based on ultrastructure. While stage I and II melanosomes are amelanotic, stage III and IV melanosomes are partially, and fully, pigmented, respectively. HRPE cultured in DMEM containing sericin displayed melanosomes of all four stages following seven days of culture, which suggests that the process of melanogenesis was ongoing.

The tight junction barrier is involved in creating a polarized epithelium and is necessary for maintaining an apical-basal concentration gradient across the RPE. Thus, as shown by TEM in the current study, sericin enabled the development of a polarized RPE, as indicated by presence of apical tight junctions and basal cell nuclei.

A20, CXCL10, C3 and CCL2 were among the top five sericin-induced genes in this study. A20 was also the top up-regulated gene in the NF-κB pathway. It is antiinflammatory and prevents NF-κB and TNF-mediated apoptosis (Beyaert et al. Biochemical pharmacology. 2000;60(8):1143-51). A20 is induced by TNF and inhibits the NF-κB pathway by de-ubiquitination and ubiquitination of the TNF receptor-interacting protein (Heyninck et al. Trends in biochemical sciences. 2005;30(1):1-4). Zinc supplementation in human AMD patients, which up-regulates A20 (Prasad et al. Free radical biology & medicine. 2004;37(8):1182-90), has been shown to be associated with decreased risk of developing advanced AMD or neo-vascular AMD during a 10-year follow-up (Chew et al. Ophthalmology. 2013;120(8):1604-11 e4). In rats, A20 (TNFAIP3) was identified as a candidate gene for development of retinopathy (Korbolina et al. BMC genomics. 2014;15 Suppl 12:S3). The CXCL10 protein is a potent inhibitor of angiogenesis and an antitumor agent causing tumor necrosis. C3 is commonly found in drusen of AMD patients, and the presence of C3 is critical for protection of the retina. Absence of C3 expression has deleterious effects on the retinal structure and leads to progressive retinal degeneration. C3 can also initiate angiogenesis, thereby opposing the anti-angiogenic effect of CXCL10. CCL2 contributes in maintaining normal RPE morphology, and lack of the gene leads to RPE cell loss and stress.

Inclusion of sericin in the culture medium leads to up-regulation of several genes related to the visual cycle, including RPE65, RDH10 and CRALBP. Retinal diseases can result from mutations or malfunction of key proteins in the visual cycle, in which the RPE serves as a critical component. The RDH10 is essential for synthesis of embryonic retinoic acid and therefore for limb, craniofacial and organ development. RPE65 is one of the key markers of RPE cells, and responsible for light-independent conversion of all-*trans*-retinyl esters into 11-*cis*-retinol. CRALBP, a marker of HRPE differentiation that is involved in retinol recycling, was increased by sericin, as demonstrated by microarray and immunofluorescence. Thus, sericin increases maturation of HRPE by both promoting melanogenesis and the visual cycle.

Viability analyses were performed to investigate whether the sericin-induced up-regulation of several inflammatory cytokines was accompanied with increased cell death. To reduce the effect of cell proliferation on cell density, the measurements of cell density were performed after 14 days of post-confluent culture with or without sericin or FBS. Sericin appeared to preserve cell density under serum-free conditions, and resulted in higher cell density than when adding either FBS or a combination of FBS and sericin to DMEM. Corroborating experiments with ETH-1 demonstrated that sericin promotes cell survival, which is in line with the downstream prediction made by IPA. Our results are further supported by a study demonstrating that sericin protects against cell death following acute serum-deprivation and studies showing that FBS can be replaced by sericin in cryopreservation media without compromising viability Sasaki et al. (Biotechnology and Applied Biochemistry 2005;42(Pt 2): 183-8) and Verdanova et al. (Biopreservation and Biobanking 2014;12(2):99-105). The increased survival of hRPE upon stimulation with sericin may be related to up-regulation of A20 (TNFAIP3), which inhibits TNF-induced apoptosis, or up-regulation of anti-oxidant genes, including the pigmentation-related gene SOD2. Downstream analysis by IPA also predicted a relationship between up-regulation of TNFAIP3 and SOD2 and increased cell viability and cell survival. In addition, a direct reactive oxygen species-scavenging effect of sericin has been reported elsewhere (Chlapanidas et al. International journal of biological macromolecules. 2013;58:47-56). In RPE, inflammatory cytokines, such as IFN-γ and TNF-α, have been shown to induce SOD2, which promotes cell survival in the presence of oxidative stress (Juel et al. PloS one. 2013;8(5):e64619). Thus, even though sericin promoted augmented expression of the inflammatory NF-κB pathway, cell survival was increased, possibly by the concomitant up-regulation of anti-apoptotic and anti-oxidant genes.

In conclusion, sericin promotes pigmentation of cultured HRPE by activating the NF-κB pathway. Sericin's potential role in culture protocols for rapid differentiation of RPE cells derived from embryonic or induced pluripotent stem cells should be investigated.

**Table 1. Taqman Assays**

| Gene symbol | Assay ID |
|---|---|
| RPE65 | Hs01071462_m1 |
| RGR | Hs00173619_m1 |
| NFKBIA | Hs00355671_g1 |
| NFKBIZ | Hs00230071_m1 |
| CSF1 | Hs00174164_m1 |
| POLR3H | Hs00978014_m1 |
| MAPRE1 | Hs01121102_g1 |

**Table 2. Differentially Expressed Genes in the NFκB-pathway in HRPE Cultured in DMEM with Sericin**

| Gene Symbol | Gene Name | Affymetrix ID | FC | P-value |
|---|---|---|---|---|
| A20 | Tumor necrosis factor, alpha-induced protein 3 | 17012946 | 10.4 | 8.7E-05 |
| Cot | Mitogen-activated protein kinase kinase kinase 8 | 16703642 | 2.5 | 1.1 E-02 |
| NF-KB2 p100 | Nuclear factor of kappa light polypeptide gene enhancer in B-cells 2 (p49/p100) | 16708623 | 2.1 | 3.3E-05 |
| BAFF | Tumor necrosis factor (ligand) superfamily, member 13b | 16776339 | 2.1 | 1.1 E-03 |
| NF-KB1 | Nuclear factor of kappa light polypeptide gene enhancer in B-cells 1 | 16969300 | 2.0 | 2.3E-04 |
| ABIN-1 | Tumor necrosis factor, alpha-induced protein 3 | 17012946 | 1.8 | 8.7E-05 |
| RelB | TNFAIP3 interacting protein 1 | 17001763 | 1.6 | 3.5E-04 |
| EGF | V-rel avian reticuloendotheliosis viral oncogene homolog B | 16863168 | 1.6 | 9.0E-04 |
| MEKK1 | Epidermal growth factor | 16969729 | 1.4 | 1.1 E-02 |
| TGF-α | Mitogen-activated protein kinase kinase kinase 1, E3 ubiquitin protein ligase | 16984945 | 1.2 | 1.5E-02 |
| TAB1 | Transforming growth factor, alpha | 16898788 | 1.1 | 4.5E-02 |

| | | | | |
|---|---|---|---|---|
| HRPE=human retinal pigment epithelial cells; DMEM=dulbecco modified eagle medium; FC=fold change | | | | |

**Table 3. Top Ten Up- and Down-regulated Genes in HRPE Cultured in DMEM with Sericin**

| Gene Symbol | Gene Name | Affymetrix ID | FC | P-value |
|---|---|---|---|---|
| CXCL10 | Chemokine (C-X-C motif) ligand 10 | 16977052 | 55.5 | 4.2E-06 |
| C3 | Complement component 3 | 16867784 | 34.7 | 1.2E-06 |
| CCL2 | Chemokine (C-C motif) ligand 2 | 16833204 | 21.6 | 9.3E-07 |
| IL6 | Interleukin 6 | 17044177 | 15.6 | 8.8E-06 |
| TNFAIP3 | Tumor necrosis factor, alpha-induced protein 3 | 17012946 | 10.4 | 8.7E-05 |
| ICAM1 | Intercellular adhesion molecule 1 | 16858137 | 9.1 | 2.0E-06 |
| PTX3 | Pentraxin 3, long | 16947357 | 8.0 | 1.4E-04 |
| ADA | Adenosine deaminase | 16919466 | 7.7 | 2.6E-06 |
| EDNRB | Endothelin receptor type B | 16779958 | 7.5 | 1.6E-05 |
| CNTNAP1 | Contactin associated protein 1 | 16834409 | 7.3 | 5.3E-06 |
| CLGN | Calmegin | 16980051 | -5.3 | 4.0E-06 |
| ADAM28 | ADAM metallopeptidase domain 28 | 17066921 | -5.5 | 2.8E-05 |
| LUZP2 | Leucine zipper protein 2 | 16722987 | -5.6 | 8.7E-05 |
| VCAN | Versican | 16986913 | -6.4 | 2.6E-05 |
| MFAP4 | Microfibrillar-associated protein 4 | 16842266 | -6.6 | 3.9E-06 |
| LRRC15 | Leucine rich repeat containing 15 | 16962911 | -6.9 | 3.2E-04 |
| ST8SIA4 | ST8 alpha-N-acetyl-neuraminide alpha-2,8-sialyltransferase 4 | 16998532 | -6.9 | 7.3E-06 |
| COL14A1 | Collagen, type XIV, alpha 1 | 17072162 | -9.2 | 4.5E-07 |
| SMOC2 | SPARC related modular calcium binding 2 | 17014798 | -9.8 | 2.8E-08 |
| GRIK3 | Glutamate receptor, ionotropic, kainate 3 | 16685330 | -16.4 | 5.3E-06 |

| | | | | |
|---|---|---|---|---|
| HRPE=human retinal pigment epithelial cells; DMEM=dulbecco modified eagle medium; FC=fold change | | | | |

**Table 4. Differentially Expressed Pigmentation-associated Genes in HRPE Cultured in DMEM with Sericin**

| Gene Symbol | Gene Name | Affymetrix ID | FC | P-value |
|---|---|---|---|---|
| IL6 | Interleukin 6 | 17044177 | 15.6 | 8.8E-06 |
| EDNRB | Endothelin receptor type B | 16779958 | 7.5 | 1.6E-05 |
| SOD2 | Superoxide dismutase 2, mitochondrial | 17025267 | 4.6 | 1.6E-05 |
| ABCA4 | ATP-binding cassette, sub-family A (ABC1), member 4 | 16689764 | 3.8 | 2.8E-05 |
| C10orf11 | Chromosome 10 open reading frame 11 | 16706350 | 3.1 | 1.1 E-05 |
| IRF1 | Interferon regulatory factor 1 | 16999776 | 3.0 | 1.0E-03 |
| PROM1 | Prominin 1 | 16974534 | 2.9 | 2.6E-05 |
| INSIG1 | Insulin induced gene 1 | 17053892 | 2.9 | 1.2E-04 |
| SLC24A5 | Solute carrier family 24, member 5 | 16800764 | 2.2 | 1.8E-04 |
| SOD3 | Superoxide dismutase 3, extracellular | 16965519 | 2.1 | 4.1 E-04 |
| TGF2 | Transglutaminase 2 | 16919158 | 2.0 | 6.9E-04 |
| HELLS | Helicase, lymphoid-specific | 16707695 | -2.0 | 3.4E-03 |

| | | | | |
|---|---|---|---|---|
| HRPE=human retinal pigment epithelial cells; DMEM=dulbecco modified eagle medium; FC=fold change | | | | |

**Table 5. Differentially Expressed Visual Cycle-associated Genes in HRPE Cultured in DMEM with Sericin**

| Gene Symbol | Gene Name | Affymetrix ID | FC | P-value |
|---|---|---|---|---|
| RPE65 | Retinal pigment epithelium-specific protein 65kDa | 16688370 | 2.8 | 2.7E-05 |
| RDH10 | Retinol dehydrogenase 10 (all-trans) | 17070013 | 1.9 | 1.7E-05 |
| RDH11 | Retinol dehydrogenase 11 (all-trans/9-cis/11-cis) | 16794064 | 1.7 | 3.7E-04 |
| RLBP1 (CRALBP) | Retinaldehyde binding protein 1 | 16813062 | 1.4 | 8.0E-04 |

| | | | | |
|---|---|---|---|---|
| HRPE=human retinal pigment epithelial cells; DMEM=dulbecco modified eagle medium; FC=fold change | | | | |

**Table 6. Verification of Affymetrix data by RT PCR**

| Gene symbol | Gene Name | RT PCR | | Affymetrix | |
|---|---|---|---|---|---|
| | | FC | P-value | FC | P-value |
| NFKBIA | Nuclear factor of kappa light polypeptide gene enhancer in B-cells inhibitor, alpha | 12.9 | 4.0E-06 | 6.9 | 2.0E-03 |
| RPE65 | Retinal pigment epithelium-specific protein 65kDa | 4.3 | 1.8E-07 | 2.8 | 2.7E-05 |
| CSF1 | Colony stimulating factor 1 (macrophage) | 6.8 | 1.8E-04 | 2.8 | 1.0E-02 |
| NFKBIZ | Nuclear factor of kappa light polypeptide gene enhancer in B-cells inhibitor, zeta | 5.1 | 2.0E-03 | 2.6 | 2.0E-02 |
| RGR | Retinal G protein coupled receptor | 3.6 | 5.4E-05 | 2.3 | 2.0E-03 |

The table shows average fold change (increase) in mRNA levels upon incubation of primary RPE cells with sericin for 12 days as compared to controls. Data are average from triplicates with MAPRE1 and POLR5H as endogenous controls. RT PCR=real time polymerase chain reaction; FC=fold change

## Claims

1. Use of sericin in the culture of retinal pigment epithelium (RPE) cells or RPE precursor cells, wherein sericin is added to the culture medium to promote pigmentation of cultured RPE cells or RPE precursor cells.

2. The use of claim 1 wherein the culture medium:
(i) is serum-free; and/or
(ii) is a chemically-defined medium; and/or
(iii) is or comprises a minimal medium; and/or
(iv) is or comprises a Minimal Essential Medium.

3. The use of claim 2 wherein the minimal medium or Minimal Essential Medium is selected from Eagles Minimal Essential Medium, Minimum Essential Medium Eagle with α modification (αMEM), Dulbecco's Modified Eagle's Medium (DMEM), DMEM/F12, IMDM, Medium 199, Medium 109, RPMI 1640, Ham F10, Ham F12, and McCoy's 5A.

4. The use of claim 3 wherein the minimal medium or Minimal Essential Medium is or comprises αMEM or DMEM with high glucose and pyruvate.

5. The use of any one of claims 1 to 4 wherein the concentration of sericin is 0.01 to 10 % (w/v), preferably 0.1 to 5, 0.1 to 4, 0.1 to 3, or 0.1 to 2 % (w/v), preferably 1 % (w/v).

6. The use of any one of claims 1 to 5 wherein the medium further comprises one or more additives selected from an antibiotic, a pH indicator, a hormone, a growth supplement, taurine, a non-essential amino acid and glutamine.

7. The use of claim 6 wherein:
(i) the antibiotic is penicillin and/or streptomycin; and/or
(ii) the pH indicator is phenol red; and/or
(iii) the hormone is hydrocortisone and/or triiodo-thyronine.

8. The use of claim 6 or 7 wherein the growth supplement is N1 medium supplement which comprises transferrin, insulin, putrescine, progesterone, selenium and biotin.

9. The use of any one of claims 6 to 8 wherein the non-essential amino acid is one or more selected from alanine, arginine, aspartic acid, cysteine, glutamic acid, glutamine, glycine, proline, serine, tyrosine, asparagine, and selenocysteine.

10. The use of any one of claims 1 to 9 wherein the medium is:
(i) αMEM comprising N1 growth supplement, taurine, triiodo-thyronine, one or more non-essential amino acids, glutamine, penicillin, streptomycin and hydrocortisone; or
(ii) DMEM with high glucose and pyruvate comprising penicillin and streptomycin.

11. The use of any one of claims 1 to 10 wherein the RPE cells are cultured for up to 60 days, preferably for up to 30, 21 or 14 days.

12. A method for the culture of RPE cells or RPE precursor cells, said method comprising culturing said cells in a medium comprising sericin, wherein the sericin promotes pigmentation of the cultured RPE cells or RPE precursor cells.

13. The method of claim 12, wherein the medium is as defined in any one of claims 2 to 10.

14. An RPE cell or population of cells produced by the use or method of any one of claims 1 to 13, wherein said RPE cell or population of cells is **characterised by** increased expression of NF-κB1 and NF-κB2, and unaltered expression of tyrosinase, relative to a control RPE cell or population of cells not cultured in sericin.

15. The RPE cell or cell population of claim 14 for use in therapy, preferably for use in treating or preventing an ophthalmological condition involving RPE, most preferably AMD.

## Patentansprüche

1. Verwendung von Sericin bei der Kultur von retinalen Pigmentepithelzellen (RPE) oder RPE-Vorläuferzellen, wobei Sericin dem Kulturmedium zugegeben wird, um die Pigmentierung von kultivierten RPE-Zellen oder RPE-Vorläuferzellen zu fördern.

2. Verwendung nach Anspruch 1, wobei das Kulturmedium:
(i) serumfrei ist; und / oder
(ii) ein chemisch definiertes Medium ist; und/oder
(iii) ein Minimalmedium ist oder umfasst; und/oder
(iv) ein Minimal Essential Medium ist oder umfasst.

3. Verwendung nach Anspruch 2, wobei das Minimalmedium oder Minimal Essential Medium ausgewählt ist aus Eagles Minimal Essential Medium, Minimum Essential Medium Eagle mit einer Modifikation (aMEM), Dulbeccos Modified Eagle's Medium (DMEM), DMEM/F12, IMDM, Medium 199, Medium 109, RPMI 1640, Ham F10, Ham F12 und McCoy's 5A.

4. Verwendung nach Anspruch 3, wobei das Minimalmedium oder das Minimal Essential Medium aMEM oder DMEM mit hohem Glucose- und Pyruvatgehalt ist oder umfasst.

5. Verwendung nach einem der Ansprüche 1 bis 4, wobei die Sericinkonzentration 0,01 bis 10% (Gew./Vol.), vorzugsweise 0,1 bis 5, 0,1 bis 4, 0,1 bis 3 oder 0,1 bis 2% (Gew./Vol.) beträgt; vorzugsweise 1% (Gew./Vol.).

6. Verwendung nach einem der Ansprüche 1 bis 5, wobei das Medium ferner eines oder mehrere Additive umfasst, die ausgewählt sind aus einem Antibiotikum, einem pH-Indikator, einem Hormon, einem Wachstumszusatz, Taurin, einer nicht essentiellen Aminosäure und Glutamin.

7. Verwendung nach Anspruch 6 wobei:
(i) das Antibiotikum Penicillin und/oder Streptomycin ist; und/oder
(ii) der pH-Indikator Phenolrot ist; und/oder
(iii) das Hormon Hydrocortison und/oder Triiodthyronin ist.

8. Verwendung nach Anspruch 6 oder 7, wobei der Wachstumszusatz ein N1-Mediumzusatz ist, der Transferrin, Insulin, Putrescin, Progesteron, Selen und Biotin umfasst.

9. Verwendung nach einem der Ansprüche 6 bis 8, wobei die nicht essentielle Aminosäure eine oder mehrere ist, die ausgewählt ist aus Alanin, Arginin, Asparaginsäure, Cystein, Glutaminsäure, Glutamin, Glycin, Prolin, Serin, Tyrosin, Asparagin und Selenocystein.

10. Verwendung nach einem der Ansprüche 1 bis 9, wobei das Medium ist:
(i) aMEM, umfassend N1-Wachstumszusatz, Taurin, Triiodthyronin, eine oder mehrere nicht essentielle Aminosäuren, Glutamin, Penicillin, Streptomycin und Hydrocortison; oder
(ii) DMEM mit hohem Glucosegehalt und Pyruvat, umfassend Penicillin und Streptomycin.

11. Verwendung nach einem der Ansprüche 1 bis 10, wobei die RPE-Zellen bis zu 60 Tage, vorzugsweise bis zu 30, 21 oder 14 Tage kultiviert werden.

12. Verfahren zur Kultur von RPE-Zellen oder RPE-Vorläuferzellen, wobei das Verfahren das Kultivieren der Zellen in einem Medium umfasst, das Sericin umfasst, wobei das Sericin die Pigmentierung der kultivierten RPE-Zellen oder RPE-Vorläuferzellen fördert.

13. Verfahren nach Anspruch 12, wobei das Medium nach einem der Ansprüche 2 bis 10 definiert ist.

14. RPE-Zelle oder Population von Zellen, hergestellt durch die Verwendung oder das Verfahren nach einem der Ansprüche 1 bis 13, wobei die RPE-Zelle oder Population von Zellen durch eine erhöhte Expression von NF-KB1 und NF-KB2 und eine unveränderte Expression von Tyrosinase gekennzeichnet ist relativ zu einer Kontroll-RPE-Zelle oder einer Population von Zellen, die nicht in Sericin kultiviert wurden.

15. RPE-Zelle oder Zellpopulation nach Anspruch 14 zur Verwendung in der Therapie, vorzugsweise zur Verwendung bei der Behandlung oder Verhinderung eines ophthalmologischen Zustands, an dem RPE beteiligt ist, am meisten bevorzugt AMD.

## Revendications

1. Utilisation de séricine dans la culture de cellules d'épithélium pigmentaire rétinien (RPE) ou de cellules précurseurs de RPE, dans laquelle la séricine est ajoutée au milieu de culture pour favoriser une pigmentation de cellules RPE ou de cellules précurseurs de RPE en culture.

2. Utilisation selon la revendication 1, dans laquelle le milieu de culture :
(i) est dépourvu de sérum ; et/ou
(ii) est un milieu défini chimiquement ; et/ou
(iii) est ou comprend un milieu minimum ; et/ou
(iv) est ou comprend un milieu essentiel minimum.

3. Utilisation selon la revendication 2, dans laquelle le milieu minimum ou milieu essentiel minimum est choisi parmi un milieu essentiel minimum de Eagle, un milieu essentiel minimum de Eagle avec modification α(αMEM), un milieu de Eagle modifié de Dulbecco (DMEM), DMEM/F12, IMDM, un milieu 199, un milieu 109, RPMI 1640, Ham F10, Ham F12 et 5A de McCoy.

4. Utilisation selon la revendication 3, dans laquelle le milieu minimum ou milieu essentiel minimum est ou comprend αMEM ou DMEM avec une haute teneur en glucose et pyruvate.

5. Utilisation selon l'une quelconque des revendications 1 à 4, dans laquelle la concentration de séricine est de 0,01 à 10 % (p/v), de préférence, de 0,1 à 5, 0,1 à 4, 0,1 à 3 ou 0,1 à 2 % (p/v), de préférence de 1 % (p/v).

6. Utilisation selon l'une quelconque des revendications 1 à 5, dans laquelle le milieu comprend en outre un ou plusieurs additifs choisis parmi un antibiotique, un indicateur de pH, une hormone, un supplément de croissance, de la taurine, un acide aminé non essentiel et de la glutamine.

7. Utilisation selon la revendication 6, dans laquelle :
(i) l'antibiotique est de la pénicilline et/ou de la streptomycine ; et/ou
(ii) l'indicateur de pH est du rouge de phénol ; et/ou
(iii) l'hormone est de l'hydrocortisone et/ou de la triiodo-thyronine.

8. Utilisation selon la revendication 6 ou 7, dans laquelle le supplément de croissance est un supplément de milieu N1 qui comprend de la transferrine, de l'insuline, de la putrescine, de la progestérone, du sélénium et de la biotine.

9. Utilisation selon l'une quelconque des revendications 6 à 8, dans laquelle l'acide aminé non essentiel est un ou plusieurs choisis parmi alanine, ariginine, acide aspartique, cystéine, acide glutamique, glutamine, glycine, proline, sérine, tyrosine, asparagine et sélénocystéine.

10. Utilisation selon l'une quelconque des revendications 1 à 9, dans laquelle le milieu est :
(i) αMEM comprenant un supplément de croissance N1, de la taurine, de la triiodo-thyronine, un ou plusieurs acides aminés non essentiels, de la glutamine, de la pénicilline, de la streptomycine et de l'hydrocortisone ; ou
(ii) DMEM avec une haute teneur en glucose et pyruvate comprenant de la pénicilline et de la streptomycine.

11. Utilisation selon l'une quelconque des revendications 1 à 10, dans laquelle les cellules RPE sont mises en culture jusqu'à 60 jours, de préférence jusqu'à 30, 21 ou 14 jours.

12. Procédé de culture de cellules RPE ou de cellules précurseurs de RPE, ledit procédé comprenant la culture desdites cellules dans un milieu comprenant de la séricine, dans lequel la séricine favorise la pigmentation des cellules RPE ou des cellules précurseurs de RPE en culture.

13. Procédé selon la revendication 12, dans lequel le milieu est tel que défini dans l'une quelconque des revendications 2 à 10.

14. Cellule ou population de cellules RPE produite grâce à l'utilisation ou au procédé selon l'une quelconque des revendications 1 à 13, dans laquelle ladite cellule ou population de cellules RPE est **caractérisée par** une expression accrue de NF-κB1 et NF-κB2, et une expression inaltérée de tyrosinase, par rapport à une cellule ou population de cellules RPE témoin non mise en culture dans de la séricine.

15. Cellule ou population de cellules RPE selon la revendication 14 destinée à une utilisation en thérapie, de préférence à une utilisation dans le traitement ou la prévention d'un trouble ophtalmologique impliquant le RPE, plus préférablement, la DMLA.
